(19) Europäisches Patentamt
European Patent Office
Office européen des brevets

(11) EP 4 623 933 A1

(12) **EUROPEAN PATENT APPLICATION**
published in accordance with Art. 153(4) EPC

(43) Date of publication:
**01.10.2025 Bulletin 2025/40**

(21) Application number: **23919510.0**

(22) Date of filing: **18.12.2023**

(51) International Patent Classification (IPC):
***A61K 47/58*** *(2017.01)* ***A61K 47/59*** *(2017.01)*
***A61K 39/395*** *(2006.01)* ***A61K 38/16*** *(2006.01)*
***A61K 38/08*** *(2019.01)* ***A61K 38/26*** *(2006.01)*
***A61K 38/29*** *(2006.01)* ***A61K 38/23*** *(2006.01)*
***A61K 38/27*** *(2006.01)* ***A61K 9/08*** *(2006.01)*
***A61P 3/10*** *(2006.01)* ***A61P 19/10*** *(2006.01)*
***A61P 37/02*** *(2006.01)*

(52) Cooperative Patent Classification (CPC):
Y02A 50/30

(86) International application number:
**PCT/CN2023/139354**

(87) International publication number:
**WO 2024/159948 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.01.2023 CN 202310044768**

(71) Applicant: **Hangzhou Tito Biotechnology Partnership Enterprise (Limited Partnership)**
**Hangzhou, Zhejiang Province 311222 (CN)**

(72) Inventors:
- **SHEN, Youqing**
  **Hangzhou, Zhejiang 310058 (CN)**
- **WEI, Qiuyu**
  **Hangzhou, Zhejiang 310058 (CN)**
- **FENG, Weiwei**
  **Hangzhou, Zhejiang 310058 (CN)**

(74) Representative: **Elzaburu S.L.P.**
**Edificio Torre de Cristal**
**Paseo de la Castellana 259 C, planta 28**
**28046 Madrid (ES)**

# (54) HIGH-PERMEABILITY PROTEIN OR POLYPEPTIDE CONJUGATE, AND USE THEREOF

(57) A high-permeability protein or polypeptide conjugate and a use thereof are provided. A high-permeability protein conjugate is produced by bonding a tertiary amine oxide group-containing polymer to a protein other than insulin. A high-permeability polypeptide conjugate is produced by bonding the tertiary amine oxide group-containing polymer to a polypeptide. The protein and polypeptide are endowed with an ability to efficiently penetrate through the stratum corneum and epithelial cells and an intratissue penetration ability. The protein and polypeptide are also endowed with excellent stability and an ability to enter cells quickly to enable the druggability of proteins acting on intracellular targets. Therefore, the high-permeability protein or polypeptide conjugate can be used to prepare a formulation for efficient transdermal administration, eye-dropping administration, or oral administration.

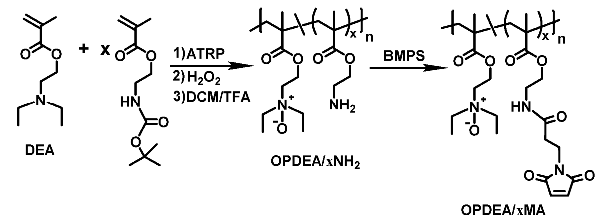

DEA
OPDEA/xNH$_2$
OPDEA/xMA

FIG. 2

EP 4 623 933 A1

## Description

## TECHNICAL FIELD

The present disclosure relates to the technical field of biomedicine, and in particular to a high-permeability protein or polypeptide conjugate and a use thereof.

## BACKGROUND

Protein-based drugs, such as monoclonal antibodies, immune antibodies, cytokines, and proteases, exhibit high activity, high specific targetability, and small toxic and side effects, and thus have become important drugs for treating cancer and other diseases. 24 monoclonal antibodies have been approved by the Food and Drug Administration (FDA) of the United States for the treatment of various tumors. For example, bevacizumab, trastuzumab, rituximab, and ramucirumab are commonly used in the clinical treatment of metastatic colon cancer, metastatic breast cancer, non-Hodgkin's lymphoma, and mutant metastatic non-small cell lung cancer. In recent years, it has been found that the monoclonal antibodies of nivolumab and pembrolizumab can activate T cells by targeting programmed cell death-1 (PD-1) to achieve immunotherapy for tumors. These two monoclonal antibodies have been approved for the clinical treatment of melanoma and squamous cell carcinoma of the lungs. Albumin-bound paclitaxel (Abraxane) is a widely used anti-tumor nanodrug. Moreover, anti-vascular endothelial growth factor (VEGF) antibodies, such as ranibizumab and conbercept, can be administered through intraocular injection to treat ocular fundus diseases caused by angiogenesis. The treatments of many diseases require the injection of immunomodulatory factors such as interleukin 12, interferon, and human granulocyte colony-stimulating factor. The deficiency of proteases can cause various diseases. For example, the deficiency of 6-phosphate dehydrogenase leads to hemolytic anemia, the deficiency of purine-metabolizing enzymes leads to hyperuricemia, gout, etc., the deficiency of tyrosinases (TYRs) leads to leukoplakia, etc., and the deficiency of growth factors leads to slow growth and development. The treatments for these diseases involve the supplementation of the corresponding active proteins.

Because proteins are macromolecules, proteins are primarily administered through subcutaneous or intravenous injection. For example, when used for treating ocular fundus diseases, drugs such as ranibizumab and conbercept cannot penetrate through the cornea to play a therapeutic role if administered as eye drops. As a result, these drugs must be administered through intraocular injection, which brings pain and risks to patients (Effects of Intravitreal Injection of Anti-Vascular Endothelial Growth Factor Drugs on Retinal Blood Circulation, Chinese Journal of Ocular Fundus Diseases, 2020, 36, 565-570). With the aid of carriers such as liposomes, oral preparations can be prepared for some proteins (CN201210390598.0, Dual-Functional Sustained-Release Protein or Polypeptide Drug Vesicle for Oral Administration and Injection and Preparation Method thereof). However, this approach does not have universality and allows low availability. In recent years, microneedle technology has been extensively studied for the transdermal delivery of proteins (CN202010259644.8, Microneedle Array for Rapid Transdermal Delivery of Protein Drugs and Preparation Method of Microneedle Array). However, a complicated preparation process is required, and issues such as infection resulting from the damage of microneedles to skin need to be solved.

Moreover, the large molecular weights (typically from several thousand to several hundred thousand Daltons) and hydrophilicity of proteins severely limit the membrane permeability and intratissue permeability of proteins. Consequently, protein molecules acting on intracellular targets are undruggable because these protein molecules fail to enter cells. The difficult diffusion and penetration of antibodies in solid tumors is a major bottleneck limiting the efficacy of antibodies (Antibody tumor penetration: transport opposed by systemic and antigen-mediated clearance. Advanced Drug Delivery Review 2008; 60: 1421-34; Tumor drug penetration measurements could be the neglected piece of the personalized cancer treatment puzzle, Clinical Pharmacology & Therapeutics 2019, 106: 148-163). Thus, enhancing the intratumoral penetration of antibodies is an important way to significantly improve the efficacy of antibodies (Enhancement of the tumor penetration of monoclonal antibody by fusion of a neuropilin-targeting peptide improves the anti-tumor efficacy, Molecular Cancer Therapy 2014, 13: 651-661).

Therefore, how to endow protein molecules with high permeability to acquire the ability to penetrate through the skin, cornea, and other epithelial tissues to achieve simple and efficient transdermal, eye-dropping, or oral administration; acquire the ability to enter cells to achieve druggability; and acquire efficient tissue permeation to improve drug efficacy has become an urgent problem to be solved.

Polypeptides are a class of compounds similar to small-molecular-weight proteins, which are produced by linking several to 100 amino acids through peptide bonds. Polypeptides mostly have a molecular weight of less than 10 kDa. Polypeptides extensively participate in and regulate the functions in organisms. As a result, polypeptides have been widely developed into various drugs, which have broad indications, high safety, and remarkable efficacy and have been extensively used for the prevention, diagnosis, and treatment of diseases such as tumors, hepatitis, diabetes, and acquired immunodeficiency syndrome. Currently, hundreds of polypeptides have been developed into clinical drugs. For

example, glucagon-like peptide 1 (GLP-1) is a hormone secreted by human intestinal L cells to promote the secretion of insulin and the proliferation of pancreatic β cells. GLP-1 can act on the stomach to inhibit the gastric evacuation. GLP-1 can also act on the central nervous system of the brain to suppress the appetite, reduce the food intake, weight, and blood pressure, and improve the blood lipid level. The current GLP-1 products for clinical practice include exenatide, lixisenatide, liraglutide, albiglutide, dulaglutide, semaglutide, pramlintide, lixisenatide, etc. There are also cytokine mimetic peptides, such as erythropoietin (EPO) and thrombopoietin (TPO); thymopentin against chronic hepatitis B; immunosuppressive peptides such as cyclosporin; anti-HIV peptides such as cobicistat and enfuvirtide; antimicrobial peptides, such as gramicidin, caspofungin, teicoplanin, and micafungin; peptides for treating osteoporosis, such as teriparatide, calcitonin salmon, and abaloparatide; anti-tumor peptides, such as degarelix, triptorelin, and mifamurtide; auxin peptides, such as sermorelin; follicle-stimulating hormones such as urofollitropin; peptides for treating acromegaly such as lanreotide and glutathione; and polypeptide vaccines.

The first disadvantage of polypeptides is that polypeptides are easily degraded by enzymes and are easily cleared by the kidneys due to small molecular weights. For example, GLP-1 is easily hydrolyzed by the DPP-4 enzyme, and the *in vivo* half-life period of GLP-1 is merely 2 min. Therefore, polypeptides need to be modified. The main modification methods include amino acid substitution, sequence modification, saturated fatty acid modification, albumin fusion, immunoglobulin Fc fragment fusion, etc. Currently, the GLP-1 receptor agonists approved by FDA include short-acting preparations such as exenatide and lixisenatide, and long-acting preparations liraglutide, exenatide, albiglutide, dulaglutide, and semaglutide (Progress on the Long-Lasting Protein and peptide Drugs, Life Sciences, 2008, 2: 258-262).

The second disadvantage of polypeptides is that most polypeptides are difficult to enter cells to play a role. The molecular sizes, polarity, hydrophilicity, and charges of polypeptides make it difficult for polypeptides (except for cell-penetrating peptides and specific receptor-targeting peptides) to penetrate through cell membranes and enter cells like small molecules. Thus, most of the polypeptides currently used target extracellular molecules, but many polypeptides have intracellular targets. The vast majority of polypeptides can hardly penetrate through physiological barriers, such as the blood-brain barrier.

The third and most significant disadvantage of polypeptide drugs is that polypeptide drugs must be administered through injection. This is mainly because polypeptides are prone to degradation and can hardly penetrate through the intestinal mucosa and skin. Therefore, the development of non-injectable polypeptide formulations has received extensive attention. Oral semaglutide, the latest GLP-1 receptor agonist approved by FDA in 2019, gets rid of the traditional subcutaneous injection mode and significantly improves the compliance of patients, but exhibits low bioavailability.

The transdermal administration of polypeptides has many advantages over the conventional administration, including convenient administration, no trauma, and no pain. The transdermal administration of polypeptides avoids the first-pass effect and the gastrointestinal tract barrier and can lead to a long-lasting, constant, and controlled plasma-drug concentration, thereby mitigating adverse reactions. Accordingly, when there is an adverse reaction or drug toxicity, the administration can be stopped in time. Therefore, the development of polypeptide preparations for transdermal administration is of great significance. The transdermal delivery methods for polypeptide drugs include chemical permeation enhancers, a variety of physical permeation enhancers, transdermal peptides, and microneedle technology (Research Progress of Polypeptides Transdermal Delivery, Chinese Journal of Hospital Pharmacy, 2018, 19: 2084-2087). However, these methods cause damage to the skin and have low transdermal efficiency.

## SUMMARY

A first objective of the present disclosure is to provide a high-permeability protein conjugate and a use thereof. That is, the present disclosure provides a technology where a tertiary amine oxide group-containing polymer is bonded to a protein to significantly improve the permeability of the protein to achieve a transdermal ability, a cell entry ability, and tissue permeability. The high-permeability protein conjugate can be used to prepare protein drugs for transdermal administration, eye-dropping administration, and oral administration, which can avoid the pain, risks, and inconvenience of injection administration and can also avoid the degradation of proteins. This technology can also give proteins an ability to enter cells quickly, and enable the druggability of proteins acting on intracellular targets. Moreover, this technology can solve the problem of penetration of protein drugs for treating tumors in solid tumors, and thus improve the anti-tumor effects of the protein drugs.

A second objective of the present disclosure is to provide a high-permeability polypeptide conjugate and a use thereof. The novel polypeptide conjugate can penetrate through the stratum corneum barrier of skin to enter the systemic blood circulation. The resistance of the tertiary amine oxide group-containing polymer to the non-specific adsorption of proteins and the adhesion of the tertiary amine oxide group-containing polymer to erythrocyte membranes can stabilize the polypeptide and prolong the blood circulation time of the polypeptide. The polypeptide conjugate can be used to prepare a transdermal formulation, an eye drop formulation, or an oral formulation.

To solve the technical problems, the present disclosure adopts the following technical solutions:
A high-permeability protein or polypeptide conjugate is provided, where a protein conjugate is produced by bonding a

tertiary amine oxide group-containing polymer to a protein other than insulin; a polypeptide conjugate is produced by bonding the tertiary amine oxide group-containing polymer to a polypeptide; and a structural formula of the protein conjugate is one of formulas (I) to (III) and a structural formula of the polypeptide conjugate is one of formulas (IV) to (VI):

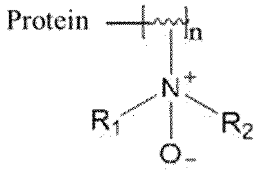

(I)

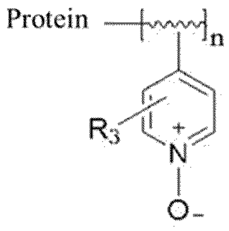

(II)

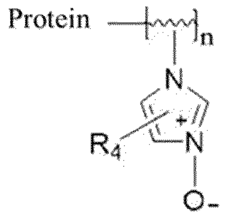

(III)

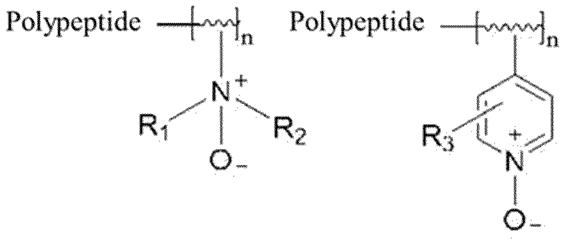

(IV) (V)

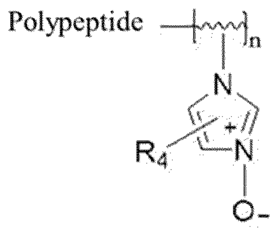

(VI)

where in the above structural formulas, $R_1$ and $R_2$ each are selected from $C_1$-$C_6$ alkyl, substituted alkyl, aryl, or substituted aryl; and $R_3$ and $R_4$ are selected from hydrogen, halogen, alkoxy, $C_1$-$C_6$ alkyl, substituted alkyl, aryl, or substituted aryl. A substituent of the substituted alkyl can be one or more of halogen, hydroxyl, alkoxy, and amino. A substituent of the substituted aryl can be one or more of halogen, hydroxyl, alkoxy, and amino. Alkyl of the substituted alkyl refers to $C_1$-$C_6$ alkyl.

Preferably, the protein is selected from one of a common protein, an antibody protein, an immunomodulatory factor protein, an enzyme protein, and a growth factor protein.

Further, the common protein includes albumin, etc.; the antibody protein includes an anti-hepatitis B virus antibody, an immunoglobulin, an immunosuppressive antibody, an anti-tumor antibody, etc.; the immunomodulatory factor protein includes an interleukin, tumor necrosis factor-alpha (TNF-$\alpha$), an interferon, a human granulocyte colony-stimulating factor, etc.; the enzyme protein includes 6-phosphate dehydrogenase, lipoprotein lipase, a purine-metabolizing enzyme, TYR, prolidase, arginase, biotinidase (BD), carboxylase, hyaluronidase, etc.; and the growth factor protein includes a growth hormone-releasing hormone (GHRH), a human growth hormone (HGH) secreted by a pituitary gland, an epidermal growth factor (EGF), a fibroblast growth factor (FGF), platelet-derived growth factor (PDGF), and somatotropin release-inhibiting hormone (SRIH) such as somatostatin.

Preferably, the polypeptide is a molecule that is produced by linking amino acids through peptide bonds and has a pharmacological action and a molecular weight of 10,000 Da or less.

The polypeptide is a natural or synthetic peptide.

Preferably, the polypeptide is selected from one of GLP-1, a cytokine mimetic peptide, an immunosuppressive peptide, an anti-inflammatory peptide, an antiviral peptide, an antimicrobial peptide, an anti-tumor peptide, a peptide for treating osteoporosis, and an auxin peptide.

Further, the GLP-1 includes exenatide, lixisenatide, liraglutide, albiglutide, dulaglutide, semaglutide, pramlintide, lixisenatide, etc.; the immunosuppressive peptide includes cyclosporin, etc.; the cytokine mimetic peptide includes EPO, TPO, etc.; the antiviral peptide includes thymopentin against chronic hepatitis B, and an anti-HIV peptide: cobicistat and enfuvirtide; the antimicrobial peptide includes caspofungin, teicoplanin, and micafungin; the peptide for treating osteoporosis includes teriparatide, calcitonin salmon, and abaloparatide; the anti-tumor peptide includes degarelix, triptorelin, and mifamurtide; and the auxin peptide includes sermorelin, urofollitropin as a follicle-stimulating hormone, lanreotide, glutathione, a polypeptide vaccine, etc.

Preferably, a functional group on the protein or the polypeptide is linked to a reactive group on a terminal chain or a side chain of the tertiary amine oxide group-containing polymer through a chemical bond. The functional group includes amino, carboxyl, hydroxyl, and sulfhydryl, and the chemical bond includes an amide bond, a urea bond, a carbamate bond, a thiourea bond, an ester bond, and an ether bond.

Preferably, a tertiary amine oxide group in the tertiary amine oxide group-containing polymer is an oxide of a saturated or unsaturated tertiary amine group, and the tertiary amine group is selected from one of N,N-dimethylamino,

N,N-diethylamino, N,N-dipropylamino, N,N-methylethylamino, N-pyrrolidinyl, N-piperidinyl, N-morpholinyl, and pyridyl.

Preferably, the tertiary amine oxide group-containing polymer is a poly(meth)acrylate, a poly(meth)acrylamide, a polymaleic anhydride, a polyamino acid, a polyester, or a polyethyleneimine.

Preferably, the tertiary amine oxide group-containing polymer has a molecular weight of 500 Da to 30,000 Da.

The tertiary amine oxide group-containing polymer can be prepared as follows: preparing a tertiary amine group-containing polymer by a polymerization method common in the polymer field, and then oxidizing a tertiary amine group into an N-oxide with an oxidant such as hydrogen peroxide or peracetic acid; or preparing a tertiary amine oxide group-containing monomer, and preparing the polymer by a conventional polymerization method; or grafting a tertiary amine oxide group-containing group to the existing polymer through a macromolecular reaction.

Further, the tertiary amine oxide group-containing polymer is selected from one of poly[2-(N-oxide-N,N-dimethylamino)ethyl methacrylate] (OPDMA), poly[2-(N-oxide-N,N-diethylamino)ethyl methacrylate] (OPDEA), [2-(N-oxide-N,N-dimethylamino)ethyl acrylate] (OPDMAA), poly[2-(N-oxide-N,N-diethylamino)ethyl acrylate] (OPDEAA), poly[N-oxide-4-vinylpyridine](OPVP), poly[glutamic acid-2-(N-oxide-N,N-dimethylamino)ethyl ester] (OPGADMe), poly[glutamic acid-2-(N-oxide-N,N-dimethylamino)acetamide] (OPGADMa), poly[glutamic acid-2-(N-oxide-N,N-diethylamino)ethyl ester] (OPGADEe), poly[glutamic acid-2-(N-oxide-N,N-diethylamino)acetamide] (OPGADEa), poly{$N^{\varepsilon}$-[2-(N-oxide-N,N-dimethylamino)ethyloxyacyl]lysine} (OPLLDMe), poly{$N^{\varepsilon}$-[2-(N-oxide-N,N-dimethylamino)ethylaminoacyl]lysine} (OPLLDMa), a polyglutamic acid (PGA) dendritic macromolecule including 2-(N-oxide-N,N-dimethylamino)ethyl ester (amide) on a surface, and a poly-L-lysine (PLL) dendritic macromolecule.

A use of the high-permeability protein or polypeptide conjugate as a raw material for preparing a transdermal formulation, an eye drop formulation, or an oral formulation is provided.

The high-permeability protein or polypeptide conjugate can be used to prepare transdermal formulations for treating tumors, rheumatoid, enzyme-deficiency disorders, gout, growth disorders caused by growth factor deficiency, etc. The high-permeability protein conjugate can also be used to prepare an eye drop formulation for treating ocular diseases, which can avoid the pain and risks caused by intraocular injection to patients.

When the polypeptide is GLP-1, the tertiary amine oxide group-containing polymer is bonded to a lysine residue of the GLP-1 or to a lysine residue introduced at a C-terminus of the GLP-1.

When the polypeptide is a semaglutide backbone, the polypeptide conjugate has a structure shown in any one of structural formulas I to IV.

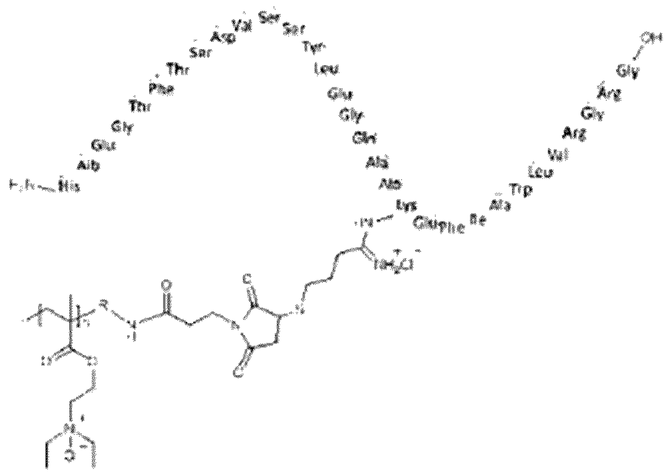

I

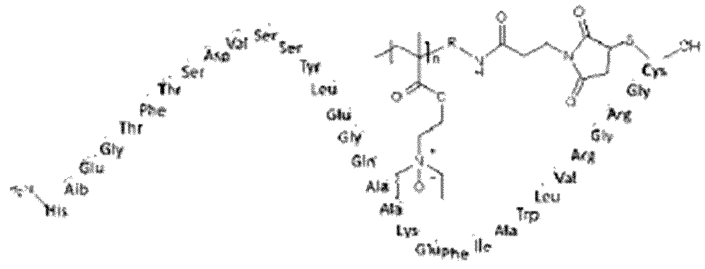

II

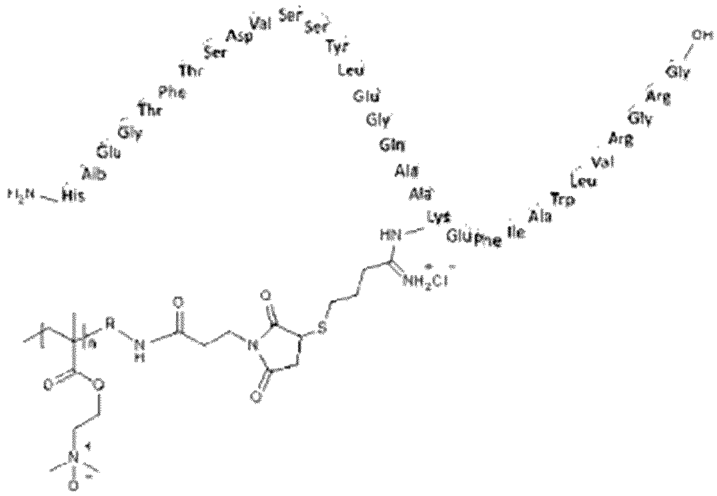

III

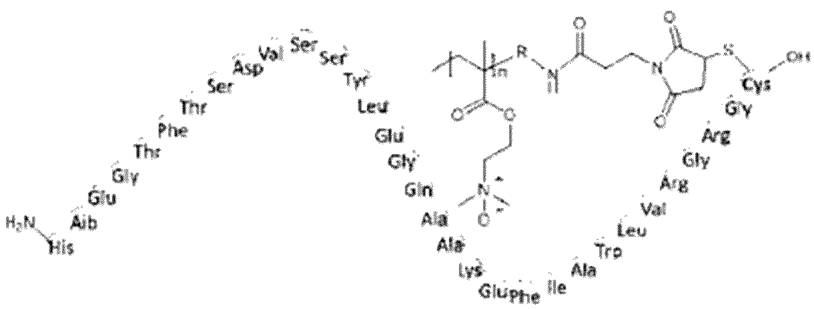

IV

A use of the high-permeability polypeptide conjugate as a raw material for preparing a transdermal formulation, an eye drop formulation, or an oral formulation is provided, including a use of the high-permeability GLP-1 conjugate as a raw material for preparing a transdermal formulation to treat obesity and type 2 diabetes.

The inventors have found through research that the tertiary amine oxide group-containing polymer provides high permeability functions: 1) The tertiary amine oxide group-containing polymer shows strong interactions with acidic lipids of the stratum corneum and phospholipid structures of cells in the skin, and can be enriched in gaps among keratinocytes and quickly penetrate through the stratum corneum to enter underlying cells. 2) This polymer can also trigger the transcytosis or rapid intercellular transport of subcutaneous cells, such that the polymer can be delivered to blood vessels and other tissues through intercellular layer-by-layer delivery. 3) The tertiary amine oxide group-containing polymer can rapidly penetrate through the epithelial cell layer. 4) The superhydrophilic neutral ion pair structure of the tertiary amine oxide group makes the polymer have an ability to penetrate through mucus (such as intestinal mucus and lung mucus).

In the preliminary studies of the inventors, the polymer is bonded to insulin, so as to enable the transdermal, oral,

and eye-dropping administration of insulin. However, with the progress of research, the inventors have further acquired unexpected and significant research results. That is, the tertiary amine oxide group-containing polymer can serve as a "locomotive" to pull a protein with a much larger molecular weight (spatial size) than insulin (molecular weight: 5,700 Da), such as albumin of 66,000 Da and an enzyme of 300,000 or more Da, to penetrate through the stratum corneum and skin tissue and enter the blood, thereby achieving the transdermal administration. As a result, the administration routes for proteins such as antibody proteins and peptide drugs can be improved. The tertiary amine oxide group-containing polymer bonded to the protein or polypeptide can stabilize the protein or polypeptide and reduce a rate at which the protein or polypeptide is degraded by a protease. The superhydrophilic poly(ion pair) characteristic of the tertiary amine oxide group-containing polymer also extends the blood circulation time of the polymer-protein or polypeptide conjugate, and prolongs the time for the protein or polypeptide to exert a potency. The polymer-protein or polypeptide conjugate also exhibits significantly-improved stability, and thus remains stable without degradation in the gastrointestinal tract after being orally administered. The polyion pair characteristic of the tertiary amine oxide group-containing polymer also endows the protein or polypeptide conjugate with an ability to penetrate through intestinal mucus and lung mucus layers and reach epithelial cells. The characteristic of the tertiary amine oxide group-containing polymer to induce the rapid intercellular transport also allows the conjugate to be quickly transported to blood and other tissues, thereby achieving the efficient absorption and efficacy exertion after administration.

The present disclosure has the following beneficial effects: The conjugate produced by bonding a tertiary amine oxide group-containing polymer to a protein can efficiently penetrate through the skin, cornea, epithelial tissue, and tumor tissue, so as to achieve the transdermal administration, eye-dropping administration, oral administration, etc. of the protein and improve the intratissue distribution and therapeutic effect of the protein drug.

The high-permeability polypeptide conjugate of the present disclosure has an ability to efficiently penetrate through the stratum corneum and epithelial tissue. Moreover, the polypeptide in the conjugate has better stability than the free polypeptide. Therefore, the high-permeability polypeptide conjugate can be used to prepare formulations for transdermal administration, eye-dropping administration, and oral administration. For example, the tertiary amine oxide group-containing polymer-GLP-1 conjugate in the present disclosure can effectively treat type 2 diabetes after being transdermally administered.

After being transdermally administered, the high-permeability protein or polypeptide conjugate of the present disclosure can be transdermally absorbed directly into capillaries and then enter the systemic circulation without destroying the skin structure and causing infections. The high-permeability protein or polypeptide conjugate has characteristics such as stable sustained release *in vivo,* long blood circulation time, and low immunogenicity, and is of great significance for the treatment of chronic diseases such as diabetes, congenital enzyme deficiency diseases, and rheumatoid requiring long-term administration and the non-injection administration-based treatment of diseases in delicate organs such as eyes.

## BRIEF DESCRIPTION OF THE DRAWINGS

FIG. 1 shows the preparation and representative structure for a tertiary amine oxide group-containing polymer with a terminal group functionalized in Examples 1 to 14, including the synthesis of OPDEA-$NH_2$ and the routes of preparing OPDEA-NHS, OPDEA-MA, and OPDEA-SH from OPDEA-$NH_2$;

FIG. 2 shows the random copolymerization of 2-(N,N-diethylamino)ethyl methacrylate (DEA) and 2-(Boc amino)ethyl methacrylate to prepare OPDEA with amino or maleimido in a side chain in Example 15;

FIG. 3 shows the representative tertiary amine oxide group-containing polyamino acids in Examples 20 to 23 (e is an ester bond and a is an amide bond);

FIG. 4 shows the functionalization of a protein or polypeptide and reaction paths for conjugating the protein or polypeptide with polymers in Examples 24 to 60;

FIG. 5 shows the bonding of amino in a lysine residue of a semaglutide backbone with OPDEA to prepare conjugates in Examples 48 to 49 (semaglutide backbone-L1OPDEA5k);

FIG. 6 shows the bonding of sulfhydryl in a cysteine residue at a C-terminus of a semaglutide backbone with OPDEA to prepare a conjugate in Example 50 (semaglutide backbone-C1OPDEA5k);

FIG. 7 shows high-performance liquid chromatography (HPLC) spectra of BSA-1OPDEA5k and OPDEA-5OPDEA5K in Examples 24 and 25 (with an electrical signal as a y-coordinate);

FIG. 8 shows the permeation testing results of a bovine serum albumin (BSA) conjugate in eyes in Example 63: laser confocal fluorescence images of eyeball sections;

FIG. 9 shows the permeation and treatment testing results of aflibercept-OPDMA conjugate groups in eyes in Examples 63 and 64, where (A) shows the laser confocal fluorescence images of eyeball sections after eye-dropping administration (0.5 mg/mL); and (B) shows the experimental results of a laser-induced choroidal neovascularization

(CNV) model administered four times a day with eye drops: the left panel shows the representative CNV spot image for each group and the right panel shows the statistics of an average area of CNV spots in each group;
FIG. 10 shows the skin permeation testing results of $^{Cy5}$BSA, $^{Cy5}$BSA-1OPDMA5k, and $^{Cy5}$BSA-1OPDMA30k in Example 65: laser confocal fluorescence images of skin sections after 4 h of skin administration;
FIG. 11 demonstrates that other protein-OPDEA conjugates can efficiently penetrate through the skin in Example 65;
FIG. 12 shows the fluorescence images of BSA-OPDEA conjugate-infiltrated tumor spheres under a laser scanning confocal microscope in Example 66;
FIG. 13 shows the fluorescence quantification for BSA-OPDEA conjugate-infiltrated tumor spheres based on the fluorescence images of the BSA-OPDEA conjugate-infiltrated tumor spheres under a laser scanning confocal microscope in Example 66 (with a fluorescence intensity as a y-coordinate);
FIG. 14 shows the comparison of effects of semaglutide-OPDEA and semaglutide backbone to promote the proliferation of islet cells;
FIG. 15 shows the effects of semaglutide-OPDEA and semaglutide backbone to promote the secretion of insulin;
FIG. 16 shows the hypoglycemic effect of semaglutide-OPDEA;
FIG. 17 shows the pharmacokinetics of semaglutide-OPDEA after transdermal administration;
FIG. 18 shows the stability of semaglutide-OPDEA under enzymolysis;
FIG. 19 shows the characterization of a uricase (UC)-OPDEA conjugate by gel permeation chromatography;
FIG. 20 shows an *in vivo* blood uric acid concentration change curve after the transdermal administration of UC-OPDEA; and
FIG. 21 shows an *in vivo* UC-OPDEA conjugate concentration change curve after transdermal administration.

## DETAILED DESCRIPTION OF THE EMBODIMENTS

The technical solutions of the present disclosure will be described in further detail below with reference to specific embodiments.

In the present disclosure, unless otherwise specified, all raw materials and devices adopted are commercially available or are commonly used in the art. All methods in the following embodiments are the conventional methods in the art, unless otherwise specified.

Example 1: Synthesis of a tertiary amine oxide group-containing polymer with a terminal group functionalized

FIG. 1A shows a reaction path for preparing a polymer with a controllable molecular weight through atom transfer radical polymerization (ATRP): According to the literature (Nature Biomedical Engineering 2021, 5, 1019-1037), the polymerization of DEA was initiated with 2-bromo-2-methyl-propionate 2'-(tert-butyloxyamido)ethyl ester as an initiator to prepare a terminal amino-protected polymer PDEA. Then the terminal amino-protected polymer PDEA was oxidized according to the literature, and the protective group was removed by the conventional trifluoroacetic acid method to produce oxidized PDEA with terminal amino, namely, OPDEA-$NH_2$. A ratio of the DEA to the initiator and a conversion rate were controlled to produce a polymer with a molecular weight of 5,100 Da.

With the above method, a molar ratio of the DEA to the initiator could be controlled to produce OPDEA-$NH_2$ with an adjustable molecular weight of 500 to 30,000. Details were shown in the following table for Examples 2 to 6.

| Example | DEA (mmoL) | Initiator (mmoL) | Conversion rate (%) | Molecular weight of a polymer (Da) |
|---|---|---|---|---|
| Example 2 | 50 | 2.5 | 40 | 1080 |
| Example 3 | 50 | 2 | 72 | 3100 |
| Example 4 | 50 | 0.5 | 64 | 10800 |
| Example 5 | 50 | 0.25 | 60 | 19800 |
| Example 6 | 50 | 0.2 | 75 | 32700 |

Similarly, under the above conditions, polymers were prepared through the polymerization of methacrylate-2-(*N,N*-dimethylamino)ethyl ester (DMA), methacrylate-2-(*N*-pyrrolidinyl)ethyl ester (PyA), and 4-vinylpyridine monomers, respectively, and then oxidized and deprotected to prepare other tertiary amine oxide group-containing polymers with terminal amino. The prepared poly[methacrylate-2-(*N,N*-dimethylamino)ethyl ester] (OPDMA-$NH_2$), poly[methacrylate-2-(*N*-pyrrolidinyl)ethyl ester] (OPPyA-$NH_2$), and poly(4-vinylpyridine) (OPVP-$NH_2$) were listed in Examples 7 to 9 (FIG. 1B).

| Example | Monomer | Polymer |
|---|---|---|
| Example 7 | Methacrylate-2-(*N,N*-dimethylamino)ethyl ester | OPDMA-$NH_2$ |
| Example 8 | 4-Vinyl(N-oxide)pyridine | OPVP-$NH_2$ |
| Example 9 | Methacrylate-2-(*N*-pyrrolidinyl)ethyl ester | OPPyA-$NH_2$ |

300 mg of the OPDEA-$NH_2$ prepared above was taken, 5 mL of a solution of 1% triethylamine in dimethyl sulfoxide (DMSO) was added, and then 3-maleimidopropionic acid hydroxysuccinimide ester (BMPS, 2.5 times a mole number of amino) was added. A reaction was allowed at room temperature for 2 h. Ultrafiltration and purification were then conducted using an ultrafiltration tube with a molecular weight of 3.5 kDa to produce OPDEA with terminal maleimido, namely, OPDEA-MA.

Similarly, OPDMA-$NH_2$ was allowed to react with molecules including the corresponding functional groups such as an isothiocyanate group, sulfhydryl, an epoxy group, and a succinimidyl ester group to produce the corresponding polymers with a terminal group functionalized. Details were shown in the following table for Examples 10 to 13.

| Example | Reactive molecule example | Terminal group of a polymer | Polymer |
|---|---|---|---|
| Example 10 | p-phenylene diisothiocyanate | Isothiocyanate | OPDMA-ITC |
| Example 11 | 2-Iminothiolane hydrochloride | Sulfhydryl | OPDMA-SH |
| Example 12 | Epichlorohydrin | Epoxy group | OPDMA-EP |
| Example 13 | Disuccinimidyl succinate | NHS-activated ester | OPDMA-NHS |

Example 14. Direct preparation of OPDEA: According to the similar conditions in Example 1, the polymerization of methacrylate-2-(*N*-oxide-*N,N*-diethylamino)ethyl ester (ODEA) in N,N-dimethylformamide (DMF) or methanol could be initiated with 2-bromo-2-methyl-propionate 2'-(tert-butyloxyamido)ethyl ester as an initiator to directly prepare the terminal amino-protected polymer OPDEA. If 2-bromo-2-methyl-propionate-NHS ester would be allowed to react with amino on a protein or polypeptide, OPDEA could be directly introduced on the protein or polypeptide through active polymerization such as ATRP under similar conditions. This method has the disadvantage of low yield, and the reaction and purification steps may lead to the inactivation of the protein or polypeptide.

Example 15. Preparation of OPDEA with a reactive group on a side chain (FIG. 2)

DEA (2.316 g), 2-(Boc amino)ethyl methacrylate, cuprous bromide (17.93 mg), 2,2'-bipyridine (39.05 mg), and ethyl 2-bromo-2-methylpropionate (24.38 mg) (molar ratio: 100:10:1:2:1) were dissolved in 4 mL of methanol, and a reaction was allowed at 45°C for 8 h. After the reaction was completed, tetrahydrofuran (THF) were added for dilution, and filtration was conducted with a short alumina column. The THF was removed through rotary evaporation. Precipitation was conducted 3 times with cold n-hexane, and then drying was conducted to produce a copolymer with a molecular weight of 10 Ka.

The polymer was added to a 30% hydrogen peroxide aqueous solution, and stirring was conducted at room temperature to allow a reaction for 5 h. With the progress of the reaction, the polymer was gradually dissolved to finally produce a clear solution. The solution was subjected to dialysis for 2 d in water by a dialysis membrane with a molecular weight cut-off of 3,500. An aqueous solution produced after the dialysis was lyophilized.

A polymer produced above was dissolved in a mixed solution of 30 mL of chloroform/5 mL of trifluoroacetic acid, and a reaction was allowed for 4 h under stirring. The trifluoroacetic acid and the solvent were removed through rotary evaporation, and then rotary evaporation was conducted 2 times with chloroform as an entrainer. Precipitation was conducted 3 times with cold n-hexane, and the solvent was removed to produce OPDEA with 10% (mol) of $NH_2$ on a side chain (OPDEA/10% $NH_2$).

OPDEA/10% $NH_2$ was dissolved in a small amount of DMSO, and 200 $\mu$L of triethylamine was added. 80 mg of BMPS (3 times a molar mass of $NH_2$) was weighed and dissolved in DMSO, and added dropwise to a polymer solution produced above. A reaction was allowed overnight under stirring. Dialysis was conducted with a solution of DMSO and water in 1:1, and lyophilization was conducted to produce OPDEA with 10% (mol) of maleimido on a side chain (OPDEA/10% MA).

An amount of 2-(Boc amino)ethyl methacrylate (x) could be controlled to adjust a content of $NH_2$ or MA in a polymer chain.

According to the method in the literature (Athanasiou V et al., Synthesis and Characterization of the Novel N$\varepsilon$-9-

Fluorenylmethoxycarbonyl-1-Lysine N-Carboxy Anhydride: Synthesis of Well-Defined Linear and Branched Polypeptides. Polymers(Basel). 2020 Nov 27; 12 (12): 2819), the ring-opening polymerization of N-carboxyanhydrides (NCAs) of amino acids was initiated with a protective amino-containing initiator, and the carboxyl or amino protective group on a side chain was removed to produce the corresponding polyamino acids with a terminal group functionalized. Details were shown in the following table for Examples 16 to 19.

| Example | Amino acid for preparing NCA | Polyamino acid | Molecular weight (Da) | Example | Bonded tertiary amine oxide group-containing molecule | Polyamino acid functionalized with a tertiary amine oxide |
|---|---|---|---|---|---|---|
| Example 16 | Glutamic acid | PGA | 9500 | 20 | 2-(N-oxide-N,N-dimethyl)ethanol or 2-(N-oxide-N,N-dimethyl)ethylamine | OPGAe or OPGAa |
| Example 17 | Aspartic acid | Polyaspartic acid (PAA) | 8900 | 21 | | OPAAe or OPAAa |
| Example 18 | Lysine | PLL | 8800 | 22 | | OPLLe or OPLLa |
| Example 19 | Serine | Polyserine (PS) | 8700 | 23 | | OPSe or OPSa |

Example 20. PGA and 2-(N-oxide-N,N-dimethyl)ethanol (amine) were subjected to an esterification or amidation reaction under the catalysis of a condensing agent to produce poly[glutamic acid 2-(N-oxide-N,N-dimethyl)ethyl ester (amide)] with a tertiary amine oxide group on a side chain (OPGAe or OPGAa, where e: ester, and a: amide) (FIG. 3A).

Example 21. PAA and 2-(N-oxide-N,N-dimethyl)ethanol (amine) were subjected to an esterification or amidation reaction under the catalysis of a condensing agent to produce poly[aspartic acid 2-(N-oxide-N,N-dimethyl)ethyl ester (amide)] with a tertiary amine oxide group (OPAAe or OPAAa).

Example 22. PLL was allowed to react with an excess amount of 1,1'-carbonyldiimidazole (1:5) overnight. The unreacted 1,1'-carbonyldiimidazole and by-products were removed. Then 2-(N-oxide-N,N-dimethyl)ethanol (amine) (1:3) was added to allow a reaction for 48 h. Purification was then conducted to produce poly{$N^{\varepsilon}$-[2-(N-oxide-N,N-dimethyl)ethoxycarbonyl lysine} (OPLLe) or poly{$N^{\varepsilon}$-[2-(N-oxide-N,N-dimethyl)ethylaminocarbonyl lysine} (OPLLa) with a tertiary amine oxide group on a side chain (FIG. 3B).

Example 23. Under the similar conditions in Example 21, PS was allowed to react with 1,1'-carbonyldiimidazole and then react with 2-(N-oxide-N,N-dimethyl)ethanol (amine) to prepare the corresponding poly{O-[2-(N-oxide-N,N-dimethyl)ethoxycarbonyl serine} (OPSe) or poly{O-[2-(N-oxide-N,N-dimethyl)ethylaminocarbonyl serine} (OPSa) with a tertiary amine oxide group on a side chain.

With the similar method, sulfhydryl, maleimido, an isothiocyanate group, and an NHS-activated ester group could be introduced into a terminal group or a side chain of a polyamino acid (FIG. 3C).

Activation of proteins and polypeptides (FIG. 4)

For amino and sulfhydryl in a protein or polypeptide, all or a part of the amino could react with a succinimidyl ester group, an isothiocyanate group, an epoxy group, etc. of a polymer or all or a part of the sulfhydryl could react with maleimido in a polymer to prepare a protein-polymer conjugate.

Amino in a protein molecule could also be further converted into sulfhydryl or maleimido. With BSA as an example, amino in the protein could be converted into sulfhydryl with 2-iminothiolane (Traut's reagent). To 50 mM phosphate buffered saline (PBS) of BSA (100 mg, 1.5 μmoL), 0.21 mg of 2-iminothiolane (1.5 μmoL) was added. Stirring was conducted at 4°C for 2 h. Ultrafiltration was conducted to produce BSA-SH. An amount of amino converted into SH in BSA could be controlled by controlling an amount of 2-iminothiolane to produce BSA-aSH.

Similarly, BSA could be allowed to react with BMPS to prepare BSA functionalized by maleimido, namely, BSA-aMA.

Example 24: Synthesis of protein-tertiary amine oxide group-containing polymer conjugates

BSA-SH (6.6 mg) was dissolved in PBS, and an equal molar mass of an N-oxide group-containing polymer OPDEA5k-MA was added. Stirring was conducted for 2 h at room temperature to produce an albumin-N-oxide group-containing polymer conjugate BSA-1OPDEA5k (SH-MA).

With BSA-MA and OPDEA-SH, the same BSA-1OPDEA5k (MA-SH) was produced.

Examples 25 to 27: Similarly, BSA-5SH, BSA-10SH, or BSA-20SH was allowed to react with a corresponding amount of OPDEA5k-MA to prepare a conjugate in which 5, 10, or 20 OPDEA chains were bonded to albumin. Alternatively, BSA-(5, 10, or 20)MA was allowed to react with OPDEA-SH to produce the same BSA-(5, 10, or 20) OPDEA5k.

Examples 28 to 30: Similarly, BSA-SH was allowed to react with an equal molar mass of OPDEA-MA having a molecular weight of 1 k, 10 k, or 30 k to prepare a conjugate in which a 1 k, 10 k, or 30 k OPDEA chain was bonded to BSA.

Examples 31 to 36: Similarly, BSA-3SH could be allowed to react with a corresponding amount of each of other polymers, OPDMA5k-MA, OPDEAA10k-MA, POPVP10k-MA, OPGADMe10k-MA, OPGADMa10k-MA, and OPLLDMe10k-MA, to prepare conjugates in which 3 corresponding polymer chains were bonded to albumin.

Similarly, a reaction between BSA-MA and OPDMA-SH could also prepare an albumin-tertiary amine oxide group-containing polymer conjugate.

Examples 37 to 47: Similarly, Abraxane-30SH, trastuzumab-2SH, Nivolumab-5SH, anti-CD47 antibody-5SH, anti-CTLA4 antibody-5SH, ranibizumab-5SH, interleukin IL-12-5SH, adenosine deaminase (ADA)-5SH, UC-5SH, and BD-5SH could be prepared and allowed to react with a corresponding SH-equivalent molar mass of OPDEA5k-MA to prepare conjugates in which a corresponding number of OPDEA chains were bonded to a protein.

The conjugates had short polypeptide chains, included few amino groups of lysine residues, and may also include sulfhydryl. Similarly, amino could be converted into sulfhydryl or maleimido. With a semaglutide backbone as an example (FIG. 5): 0.21 mg of 2-iminothiolane (1.5 μmoL) was added to 50 mM PBS of semaglutide backbone-$NH_2$ (with amino groups of lysine residues retained) (1.5 μmoL). Stirring was conducted at 4°C for 2 h. Ultrafiltration was conducted to produce semaglutide backbone-SH.

Example 48: The semaglutide backbone-SH was allowed to react with an equal molar mass of OPDEA5k-MA for 2 h to produce a semaglutide backbone-L1OPDEA5k conjugate in which an OPDEA chain was linked to lysine in the middle of a peptide chain.

Example 49: The semaglutide backbone-L1OPDEA5k conjugate could also be prepared by allowing amino of the semaglutide backbone to directly react with an equal molar mass of OPDEA including a terminal NHS-activated ester group (OPDEA-NHS).

Example 50: Cysteine was introduced at a C-terminus during the synthesis of a semaglutide backbone. SH of a cysteine residue was allowed to react with an equal molar mass of OPDEA5k-MA for 2 h to prepare a semaglutide backbone-C1OPDEA5k conjugate in which an OPDEA chain was linked at a C-terminus (FIG. 6).

Example 51: With the method in Example 47, semaglutide-$NH_2$ (with amino groups of lysine residues retained) was allowed to react with an equal molar mass of 2-iminothiolane (1.5 μmoL) and OPDEA5k-MA to produce a semaglutide-L1OPDEA5k conjugate in which an OPDEA chain was linked to lysine in the middle of a peptide chain.

Examples 52 to 53: With the method in Example 47 or 48, exenatide was allowed to react with an equal molar mass or a 2-fold mole number of OPDEA-SH or OPDEA-MA to prepare an exenatide conjugate with 1 or 2 OPDEA chains bonded.

Example 54: A terminal group of gramicidin was formamido, which needed to be converted into amino for bonding. 400 mg of gramicidin was weighed and dissolved in 50 mL of methanol, 15 mL of a 16% hydrochloric acid aqueous solution was added, and stirring was conducted to allow a reaction for 4 h. Then the methanol was removed through rotary evaporation, and then rotary evaporation was conducted once with methanol as an entrainer. Washing was conducted once with a 1 M NaOH solution and 3 times with deionized water to produce gramicidin with terminal amino (gramicidin-$NH_2$).

56.5 mg of the gramicidin-$NH_2$ was weighed and dissolved in DMSO, and bubbling was conducted for 15 min to remove the air. 4.3 mg of a Traut's reagent was weighed and dissolved in DMSO, and then added to a solution produced above. A reaction was allowed for 6 h under stirring to produce gramicidin-SH.

Gramicidin-SH was allowed to react with an equal molar mass of OPDEA5k-MA to produce a linear gramicidin-1OPDEA5k conjugate.

Example 55. The OPDEA/10% MA in Example 15 was allowed to react with gramicidin-SH (a molar ratio of MA to SH was 1:1) to prepare a comb-like conjugate with OPDEA/10% gramicidin as a side chain.

Examples 56 to 60. Similarly, thymopentin, teriparatide, calcitonin salmon, and sermorelin each were allowed to react with an equal molar mass of a Traut's reagent, and then OPDEA5k-MA was added to produce the corresponding single-chain conjugates. Teriparatide, calcitonin salmon, and sermorelin each were allowed to react with a 2-fold molar mass of a Traut's reagent, and then a 2-fold amount of OPDEA5k-MA was added to produce the corresponding double-OPDEA-chain conjugates.

Examples 24 to 60 Synthesized representative conjugates

| | Polymer and molecular weight (KDa) thereof | Protein or polypeptide | Number of polymer chains bonded by each protein or polypeptide | Conjugate |
|---|---|---|---|---|
| Example 24 | OPDEA 5 | Albumin BSA | 1 | BSA-1OPDEA5K (SH-MA) |
| | | Albumin BSA | 1 | BSA-1OPDEA5K (MA-SH) |
| Example 25 | OPDEA 5 | Albumin BSA | 5 | BSA-5OPDEA5K |
| Example 26 | OPDEA5 | Albumin BSA | 10 | BSA-10OPDEA5K |
| Example 27 | OPDEA5 | Albumin BSA | 20 | BSA-20OPDEA5K |
| Example 28 | OPDEA 1 | Albumin BSA | 1 | BSA-$_1$OPDEA Ik |
| Example 29 | OPDEA 10 | Albumin BSA | 1 | BSA-$_1$OPDEA $_1$OK |
| Example 30 | OPDEA 30 | Albumin BSA | 1 | BSA-1OPDEA30K |
| Example 31 | OPDMA 5 | Albumin BSA | 3 | BSA-1OPDMA3K |
| Example 32 | OPDEAA 10 | Albumin BSA | 3 | BSA-3OPDEAA10k |
| Example 33 | POPVP 10 | Albumin BSA | 3 | BSA-3POPVP10k |
| Example 34 | OPGADMe 10 | Albumin BSA | 3 | BSA-3OPGADMe10k |
| Example 35 | OPGADMa 10 | Albumin BSA | 3 | BSA-3OPGADMa10k |
| Example 36 | OPLLDMe 10 | Albumin BSA | 3 | BSA-3OPLLDMe10k |
| Example 37 | OPDEA 30 | Abraxane-30SH | 30 | Abraxane-$_3$OOPDEA5k |
| Example 38 | OPDEA 5 | Trastuzumab | 2 | Trastuzumab-2OPDEA5K |
| Example 39 | OPDEA 5 | Anti-PD-L1 antibody Nivolumab | 5 | Nivolumab-5OPDEA5K |
| Example 40 | OPDEA 5 | Anti-CD47 anti-body | 5 | Anti-CD47 antibo-dy-5OPDEA5K |
| Example 41 | OPDEA 5 | Anti-CTLA4 anti-body | 5 | Anti-CTLA$_4$ antibo-dy-5OPDEA5K |
| Example 42 | OPDEA 5 | Aflibercept | 5 | Aflibercept-SOPDEASK |
| Example 43 | OPDEA 5 | Interleukin 12 (IL-12) | 5 | IL-12-5OPDEA5K |
| Example 44 | OPDEA 5 | ADA | 5 | ADA-5OPDEA5K |

(continued)

| | Polymer and molecular weight (KDa) thereof | Protein or polypeptide | Number of polymer chains bonded by each protein or polypeptide | Conjugate |
|---|---|---|---|---|
| Example 45 | OPDEA 5 | UC | 5 | DC-5OPDEA5K |
| Example 46 | OPDEA 5 | TYR | 5 | TYR-5OPDEA5K |
| Example 47 | OPDEA 5 | BD | 5 | BD-5OPDEA5K |
| Example 48 | OPDEA 5 | Semaglutide back-bone-$NH_2$ | 1 | Semaglutide backbone-L1OPDEA5K |
| Example 49 | OPDEA 5 | Semaglutide back-bone-$NH_2$ (direct reaction) | 1 | Semaglutide backbone-L1OPDEA5K |
| Example 50 | OPDEA 5 | Semaglutide backbone | 1 | Semaglutide backbone-C1OPDEA5K |
| Example 51 | OPDEA5 | Semaglutide-NH2 (converted into SH) | 1 | Semaglutide-L1OPDEA5K |
| Example 52 | OPDEA 5 | Exenatide | 1 | Exenatide-1OPDMA5K |
| Example 53 | OPDEA 5 | Exenatide | 2 | Exenatide-2OPDEA5k |
| Example 54 | OPDEA 5 | Gramicidin | 1 | Gramicidin-$_1$OPDEA5k |
| Example 55 | OPDEA/10%MA | Gramicidin | 10% OPDEA monomer (mol) | OPDEA/10% gramicidin |
| Example 56 | OPDEA 5 | Thymopentin 1 | 1 | Thymopentin-1OPDEA5k |
| Example 57 | OPDEA 5 | Teriparatide | 1 | Teriparatide-1OPDEA5k |
| Example 58 | OPDEA 5 | Teriparatide 3 | 2 | Teriparatide-2OPDEA5k |
| Example 59 | OPDEA5 | Calcitonin salmon 2 | 1 | Calcitonin salmon-$_1$OP-DEA 5k |
| Example 60 | OPDEA5 | Auxin peptide sermorelin | 2 | Sermorelin-2OPDEA5k |

Example 61. Characterization of conjugates: The generation of conjugates was analyzed by HPLC with a 20 mM phosphate buffer of pH 7.0 as a mobile phase, a flow rate of 0.6 mL/min, and a column model of TSKgel G3000SWXL7.8*300. FIG. 7 shows HPLC spectra of BSA bonded with different numbers of OPDEA chains, and indicates the generation of conjugates.

Example 62 Fluorescently-labeled $^{Cy5}$BSA and $^{Cy5}$BSA-OPDMA

6.6 mg of BSA (0.1 μmmol) was dissolved in 1 mL of PBS at pH 7.4, 66 μg of a fluorescent dye Cy5-NHS was added, and stirring was conducted for 6 h at room temperature. Ultrafiltration-washing was repeated multiple times to produce fluorescently-labeled $^{Cy5}$BSA. The conjugate $^{Cy5}$BSA-OPDMA and other protein conjugates were labeled by the same method.

Example 63 Intraocular permeation experiment

Upper and lower eyelids of each mouse were gently pulled open with a cotton swab. Solutions of $^{Cy5}$BSA and $^{Cy5}$BSA-OPDMA in PBS each were dropped to a conjunctival sac of a mouse, and a few seconds later, eyelids were slowly closed. At 4 h after the eye-dropping administration, eyeballs were immediately collected, washed three times with PBS, fixed overnight in an FAS eyeball fixative solution, and then transferred to a 30% sucrose solution for dehydration. When sank to a bottom of the solution, eyeballs were carefully taken out, embedded with an optimal cutting temperature (OCT) embedding medium, and sectioned by a freezing microtome to produce 10 $\mu$m-thick eyeball sections. The eyeball sections were stained with a 4',6-diamidino-2-phenylindole (DAPI) dye, and finally the fluorescence distributions of each solution in eyeballs and retinas were observed under a laser scanning confocal microscope. The fluorescence distribution inside a retina of a mouse eyeball was analyzed with Image J.

According to the results of FIG. 8, $^{Cy5}$BSA only adhered to a surface of an eyeball. However, $^{Cy5}$BSA-OPDMA could penetrate into intraocular cells, especially a retina in a posterior segment of an eye, and had a high degree of fluorescence coincidence with intraocular cells, which could also be verified according to a colocalization coefficient of image analysis.

As shown in FIG. 9, aflibercept-OPDMA also had a similar intraocular permeation ability, and could indeed exert a therapeutic effect after being dropped to eyes. It indicates that a protein molecule bonded with a tertiary amine oxide group-containing polymer can penetrate in eyes and reach the deep retina, thereby overcoming the intraocular barrier and achieving the effect of treating posterior segment eye diseases through eye-dropping administration.

Example 64. Administration of aflibercept-OPDMA as eye drops to treat CNV

40 female C57BL/6J mice at 6 to 8 weeks were selected, and the anterior segments of both eyes and the fundus were examined to be normal before the experiment. An unilateral eye of each mouse was selected and subjected to laser photocoagulation to establish a CNV model, where a corresponding contralateral eye was not treated. Then treated mice were randomly divided into a PBS group, an aflibercept-OPDMA conjugate group, an OPDMA + aflibercept mixture group, and an aflibercept intravitreal injection group. Immediately after the laser photocoagulation, mice in each group were administered with eye drops at a dose of (2 $\mu$L, 10 mg/mL of aflibercept) four times a day consecutively for 7 d. For the injection group, a dose was 10 $\mu$g/mouse, and the injection was conducted merely once during the experiment.

After the laser photocoagulation, the fundus examination and the fundus fluorescein angiography (FFA) were conducted at specific time points (day 1, day 3, day 5, and day 7) to determine a generation rate of CNV and a neovascularization area, thereby determining a treatment effect of each group. As shown in FIG. 9, there was a prominent therapeutic effect in the aflibercept-OPDMA conjugate group with eye-dropping administration, but the simple mixing of the polymer and aflibercept did not allow a therapeutic effect.

Example 65 Skin permeation experiment

C57BL/6 mice each with a body weight of 20 g were selected and randomly divided into four groups. The hair on the back of each mouse was removed with a depilatory cream. After 24 h of raising, a silicone diffusion cell was fixed on the back, and Cy5-labeled $^{Cy5}$BSA and $^{Cy5}$BSA-OPDMA were added at equal amounts. 4 h after the administration, the dorsal skin of each mouse was carefully collected, rinsed 3 times with PBS, and sectioned by a freezing microtome to produce 20 $\mu$m-thick skin sections. A skin permeation effect of each solution was observed under a laser scanning confocal microscope.

As shown in FIG. 10, a faint red fluorescence was observed merely in a surface layer of the skin treated with Cy5-labeled $^{Cy5}$BSA. On the contrary, in the skin treated with $^{Cy5}$BSA-OPDMA, there was a strong fluorescence on the surface, the fluorescence could penetrate into the deep skin, and a red fluorescence could also be observed even in the dermis. It can be known that the tertiary amine oxide group-containing polymer can promote the transdermal absorption capacity of a protein drug, and improve the transdermal performance and efficacy of such a biological macromolecular drug. FIG. 11 shows that these other protein conjugates can efficiently penetrate through the skin and can be concentrated in large quantities in and under the skin, while the protein (aflibercept) itself cannot permeate into the skin.

Example 66 Permeation in tumor spheres

The permeability of albumin and a conjugate BSA-OPDMA thereof was verified with multicellular tumor spheres. 1.0 mL of DMEM and the prepared CT26 tumor spheres were added to a confocal imaging dish, and then 20 $\mu$L of $^{Cy5}$BSA-OPDMA or $^{Cy5}$BSA was added (where a concentration of BSA was 20 $\mu$g/mL). After being incubated for 2 h, tumor spheres were subjected to laser scanning confocal microscopy imaging every 20 $\mu$m along a Z-axis. Results were shown in FIG. 12. It could be observed that $^{Cy5}$BSA could only reach the edges of tumor spheres, while $^{Cy5}$BSA-OPDMA presented a strong

red fluorescence within tumor spheres, indicating significantly-enhanced permeability. Moreover, the more the OPDMA bonded, the stronger the promotion on the permeability of BSA. A molecular weight of OPDEA also exhibited an impact on the permeation in tumor spheres. OPDEA with a molecular weight of 10 k could more efficiently promote the penetration of $^{Cy5}$BSA than OPDEA with a molecular weight of 5 k. This conclusion was further confirmed by the quantitative fluorescence data in FIG. 13.

Example 67: *In vitro* activity testing of semaglutide backbone-1OPDEA5k

Rat insulinoma cells (INS1) were co-cultured with the semaglutide backbone-1OPDEA5k *in vitro* to determine whether the semaglutide backbone-1OPDEA5k could promote the proliferation of islet cells and the secretion of insulin and investigate a physiological activity of the semaglutide backbone-1OPDEA5k. Specific operations were as follows: An experiment to determine an effect of the semaglutide backbone-1OPDEA5k to promote the proliferation of islet cells: 160 μM, 40 μM, 20 μM, 10 μM, and 5 μM I solutions and semaglutide backbone solutions were prepared and then diluted 10-fold with a Krebs-Ringer Bicarbonate (KRB) buffer. INS1 cells were inoculated at a cell density of $1 \times 10^5$ cells/mL into a 96-well plate, and incubated for 24 h in an incubator at 5% $CO_2$, 37°C, and a saturated humidity. A medium was removed, 100 μL of a KRB buffer including 11 mM of glucose was added to each well, and incubation was conducted for 24 h. The buffer was removed, 100 μL of a KRB buffer including 5 mM of glucose was added to each well, and incubation was conducted for 24 h. The buffer was removed, 100 μL of a KRB buffer without glucose was added to each well, and incubation was further conducted for 2 h. The buffer was removed, and rinsing was conducted three times with a KRB buffer. 110 μL of a CCK8 medium solution was added to each well, incubation was conducted for 2 h, and an absorbance value at 450 nm was then determined by a microplate reader. 100 μL of each of the semaglutide backbone-1OPDEA5k or semaglutide backbone solutions at different concentrations was added based on a corresponding group. 90 μL of a KRB buffer and 10 μL of an aqueous solution including monosodium phosphate and propylene glycol were adopted as blank controls. After the administration, incubation was conducted in an incubator for 2 h. The buffer was removed, and rinsing was conducted three times with a KRB buffer. 110 μL of a CCK8 medium solution was added to each well, incubation was conducted for 2 h, and an absorbance value at 450 nm was then determined by a microplate reader. When a number of cells before administration was set as $n_{before}$ and a number of cells after administration was set as $n_{after}$, a proliferation rate of cells was calculated as follows: $\frac{n_{after}-n_{before}}{n_{after}} \times 100\%$.

Results were shown in FIG. 14. Both the semaglutide backbone-1OPDEA5k and the semaglutide backbone had an ability to promote the growth of islet cells, and with the increase of a concentration, the ability to promote the growth of cells gradually decreased. The semaglutide backbone-1OPDEA5k exhibited a significantly better overall effect than the semaglutide backbone, indicating that the semaglutide backbone-1OPDEA5k still exhibited a high biological activity in promoting the proliferation of islet cells.

Example 68: An experiment to determine an effect of the semaglutide backbone-1OPDEA5k to promote the secretion of insulin by islet cells: 160 μM, 40 μM, 20 μM, 10 μM, and 5 μM semaglutide backbone-1OPDEA5k and semaglutide backbone solutions were prepared and then diluted 10-fold with a KRB buffer. 100 μL of INS1 cells was added to each well of a 96-well plate at a cell density of $1 \times 10^5$ cells/mL, and incubated for 12 h in an incubator at 5% $CO_2$, 37°C, and a saturated humidity. A medium was removed, and rinsing was conducted three times with a KRB buffer. 110 μL of a CCK8 medium solution was added to each well, incubation was conducted for 2 h, and an absorbance value at 450 nm was then determined by a microplate reader. Rinsing was conducted three times with a KRB buffer. 100 μL of a KRB buffer including 10 mM of glucose was added to each well, and incubation was conducted for 1 h in an incubator at 5% $CO_2$, 37°C, and a saturated humidity. Rinsing was conducted three times with a KRB buffer. 100 μL of each of the I or semaglutide solutions with different concentrations prepared previously or a blank control was added to the plate, and incubation was conducted in an incubator for 1 h. A supernatant was collected from each well. A secretion of insulin was determined with a rat insulin enzyme-linked immunoassay kit. An absorbance value at 450 nm was determined by a microplate reader. Accordingly, an insulin secretion per unit number of INS1 cells was calculated. Results were shown in FIG. 15. The semaglutide backbone-1OPDEA5k exhibited the same activity as the original liraglutide. An insulin release-promoting effect of the semaglutide backbone-1OPDEA5k at a concentration of 4 μM had surpassed the maximum insulin release-promoting effect that could be achieved by the semaglutide backbone.

Example 69: *In vivo* circulation time and hypoglycemic effect of semaglutide backbone-1OPDEA5k

Because db/db mice have similar symptoms to human type 2 diabetes, including polydipsia, polyphagia, polyuria, obesity, hyperglycemia, dyslipidemia, etc. Thus, db/db mice are adopted as a type 2 diabetes animal model in this study. 15 healthy db/db mice were selected and randomly divided into three groups A, B, and C. The group A was an experimental group with the transdermal administration of semaglutide backbone-1OPDEA5k, the group B was a group

with the subcutaneous injection of semaglutide backbone, and the group C was an experimental group with the transdermal administration of a blank control. The experimental mice were fasted for 6 h and then intraperitoneally injected with a glucose solution at 2 g/kg. Blood was collected from orbits of mice at different time points. A plasma-drug concentration and a blood glucose change trend after administration were determined by enzyme-linked immunosorbent assay and a blood glucose meter to acquire the pharmacokinetic and pharmacodynamic data. According to the data in FIG. 16 and FIG. 17, the semaglutide backbone itself cannot penetrate through the skin and enter the blood, while the semaglutide backbone-1OPDEA5k can quickly penetrate through the skin and enter the blood, has a longer half-life period than the semaglutide backbone, and can significantly reduce the blood glucose level *in vivo.*

Example 70 Stability experiment of a conjugate

The semaglutide backbone-OPDEA5k was dissolved in PBS at 20 μg/mL, and dipeptidyl peptidase 4 (DPP-4) or neutral endopeptidase (NEP) was added at different concentrations. A concentration of the semaglutide backbone-OPDEA5k was determined at different time points to evaluate the stability of the semaglutide backbone-OPDEA5k. As shown in FIG. 18, the semaglutide backbone-1OPDEA5k exhibited significantly-higher stability than the semaglutide backbone itself. Similarly, the protein conjugate also exhibited prominent stability for the corresponding enzymes.

Example 71 Evaluation of *in vivo* efficacy of a UC-OPDEA conjugate in mice after transdermal administration

Characterization of the UC-OPDEA conjugate by gel permeation chromatography

A 0.5 M sodium nitrate solution was adopted as a mobile phase. The separation was conducted by a gel chromatographic column PL-aquagel-OH (8 μm, 300 × 7.5 mm). The detection was conducted by a photodiode array detector. The UC and UC-OPDEA conjugate were injected separately, with an injection volume of 20 μL. Chromatograms were recorded.

Experimental results were shown in FIG. 19. According to the chromatogram results, a peak of UC appeared at about 9.2 min, and a peak of OPDEA-modified UC appeared at about 8.2 min, which was one minute earlier. This is because the modification with the OPDEA polymer led to an increased molecular weight of the conjugate. The advanced peak time after the separation with the gel chromatographic column indicated that the UC-OPDEA conjugate was successfully prepared.

Determination of an average modification degree and an *in vitro* enzymatic activity of the UC-OPDEA conjugate

Determination of the average modification degree of the UC-OPDEA conjugate

The average modification degree of the UC-OPDEA conjugate was determined by an Ellman's reagent method. A product of a reaction of an Ellman's reagent with free sulfhydryl had specific absorption at 412 nm. Under specific conditions, an absorbance value was proportional to a sulfhydryl concentration. UC modified with the TR reagent had free sulfhydryl. Numbers of sulfhydryl groups in UC and UC modified with OPDEA were determined by the Ellman's reagent. The average modification degree of the UC-OPDEA conjugate was determined.

Determination of the *in vitro* enzymatic activity of the UC-OPDEA conjugate

The *in vitro* enzymatic activity of the UC-OPDEA conjugate was determined by a spectrophotometer. A maximum ultraviolet absorption wavelength of uric acid as a substrate for UC was 293 nm. A maximum ultraviolet absorption wavelength of allantoin as a product was 224 nm. An absorption value of uric acid at 293 nm was proportional to a concentration in a specified concentration range. Uric acid could be quantified by a spectrophotometer. A specific process was as follows: An ultraviolet-visible spectrophotometer was turned on, a wavelength was adjusted to 293 nm, and a water-bath circulation system of the spectrophotometer was turned on to maintain a temperature at 37°C. A sodium tetraborate buffer was adopted as a blank control. Zero-point calibration was conducted. 2.95 mL of a substrate reaction solution (0.1 mol/L of sodium tetraborate, 100 μmol/L of uric acid, pH 9.5, preheated at 37°C) was taken and placed in a quartz cuvette, then 50 μL of a test sample was immediately added, and thorough mixing was allowed. An absorption value was determined at 293 nm. A change in absorbance at 293 nm was continuously determined. A concentration of uric acid during its degradation by uricase was calculated according to $C = A/\varepsilon L$ (where A represents an absorbance value of a specific concentration of uric acid at 293 nm, ε represents a molar extinction coefficient of uric acid, L represents an optical path of the cuvette, and C represents a molar concentration of uric acid), and an enzymatic activity was calculated. Definition of the enzymatic activity: An enzyme amount required to convert 1 μmol of uric acid into allantoin per minute at the optimal reaction temperature of 37°C and the optimal reaction pH of 8.5 was defined as one activity unit (U).

Table 1 Average modification degree and *in vitro* enzymatic activity of the UC-OPDEA conjugate

| | Enzymatic activity | Average modification |
|---|---|---|
| UC | 20 U/mg | 0 |
| UC-OPDEA conjugate | 15U/mg | 10 |
| Note: The average modification degree refers to an amount of an OPDEA polymer bound to each UC monomer. | | |

The data in Table 1 showed that, after UC was modified with 10 tertiary amine oxide structure-containing polymers on average, the enzymatic activity did not decrease significantly, and the enzymatic activity of UC was still retained. As a result, the modified UC had an ability to reduce a blood uric acid concentration *in vivo.*

Example 72 Evaluation of *in vivo* efficacy of a UC-OPDEA conjugate in mice after transdermal administration

A hyperuricemia model was constructed by inducing male Balb/c mice through the subcutaneous injection of potassium oxonate in combination with the intragastric administration of a hypoxanthine soluble starch feed to evaluate a therapeutic effect of a tertiary amine N-oxide structure-containing polymer-UC conjugate for hyperuricemia in mice. 20 model mice were selected and randomly divided into 4 groups, including a model group, a UC-OPDEA (Example 45) transdermal administration group (200 U/kg), and a UC transdermal administration group (200 U/kg), with 5 mice in each group. 5 normal mice were additionally selected as a blank control group. The experimental modeling was conducted for 4 weeks. 4 weeks after the modeling, the transdermal administration was started. Blood was collected from orbital veins at 1 h, 4 h, 12 h, 36 h, 60 h, 84 h, and 108 h after each administration, and changes of blood uric acid and UC concentrations in mice were detected with a UC/uric acid test kit.

According to the data in FIG. 20, 4 weeks after the modeling, the model group had a significantly-higher blood uric acid concentration than the blank control group, indicating that the model was successfully established. In the transdermal administration experiment, the UC group had a comparable blood uric acid concentration to the model group, indicating that UC not modified with a tertiary amine oxide structure-containing polymer could not penetrate through the skin and enter the blood. However, the tertiary amine oxide structure-containing polymer-UC conjugate could penetrate through the skin and enter the blood, exhibited a prominent blood uric acid-lowering effect within 108 h *in vivo,* and could control a blood uric acid concentration *in vivo* in the short term. According to the data in FIG. 21, UC modified with a tertiary amine oxide structure-containing polymer was released at a specified rate *in vivo* after penetrating through the skin. A blood concentration of the tertiary amine oxide structure-containing polymer-UC conjugate *in vivo* reached a maximum within 12 h, and then slowly decreased at a specified rate, indicating a specified sustained release effect.

The above examples are merely preferred solutions of the present disclosure and are not intended to limit the present disclosure in any form, and other variations and modifications may be made without departing from the technical solutions of the present disclosure as set forth in the appended claims.

## Claims

1. A high-permeability protein or polypeptide conjugate, wherein the high-permeability protein conjugate is produced by bonding a tertiary amine oxide group-containing polymer to a protein other than insulin; the high-permeability polypeptide conjugate is produced by bonding the tertiary amine oxide group-containing polymer to a polypeptide; and a structural formula of the high-permeability protein conjugate is one of formulas (I) to (III) and a structural formula of the high-permeability polypeptide conjugate is one of formulas (IV) to (VI):

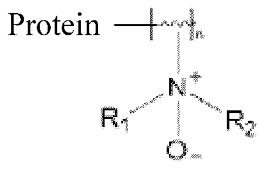
(I)

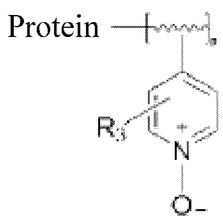
(II)

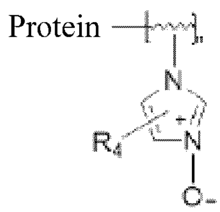
(III)

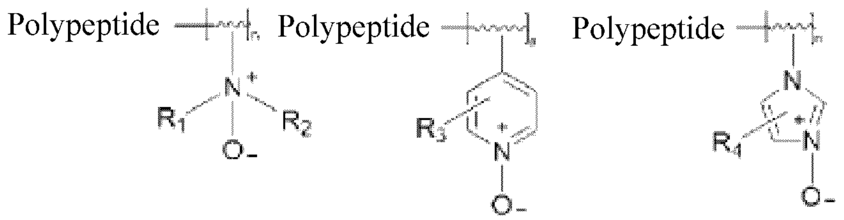

(IV) (V) (VI)

wherein in the formulas (I) to (VI), $R_1$ and $R_2$ each are selected from $C_1$-$C_6$ alkyl, substituted alkyl, aryl, or substituted aryl; and $R_3$ and $R_4$ are selected from hydrogen, halogen, alkoxy, $C_1$-$C_6$ alkyl, substituted alkyl, aryl, or substituted aryl.

**2.** The high-permeability protein or polypeptide conjugate according to claim 1, wherein the protein is selected from one of a common protein, an antibody protein, an immunomodulatory factor protein, an enzyme protein, and a growth factor protein.

**3.** The high-permeability protein or polypeptide conjugate according to claim 2, wherein the common protein comprises albumin; the antibody protein comprises an anti-hepatitis B virus antibody, an immunoglobulin, an immunosuppressive antibody, and an anti-tumor antibody; the immunomodulatory factor protein comprises an interleukin, an interferon, and a human granulocyte colony-stimulating factor; the enzyme protein comprises glucose-6-phosphate dehydrogenase, lipoprotein lipase, a purine-metabolizing enzyme, tyrosinase (TYR), prolidase, arginase, biotinidase (BD), hyaluronidase, and carboxylase; and the growth factor protein comprises a growth hormone-releasing hormone (GHRH), a human growth hormone (HGH) secreted by a pituitary gland, an epidermal growth factor (EGF), a fibroblast growth factor (FGF), platelet-derived growth factor (PDGF), and somatotropin release-inhibiting hormone (SRIH).

**4.** The high-permeability protein or polypeptide conjugate according to claim 1, wherein the polypeptide is produced by linking amino acids through peptide bonds and has a pharmacological action and a molecular weight of 10,000 Da or less.

**5.** The high-permeability protein or polypeptide conjugate according to claim 1, wherein the polypeptide is a natural or synthetic peptide, and the polypeptide is selected from one of a glucagon-like peptide 1 (GLP-1), a cytokine mimetic peptide, an immunosuppressive peptide, an anti-inflammatory peptide, an antiviral peptide, an antimicrobial peptide, an anti-tumor peptide, a peptide for treating osteoporosis, and an auxin peptide.

**6.** The high-permeability protein or polypeptide conjugate according to claim 5, wherein the GLP-1 comprises exenatide, lixisenatide, liraglutide, albiglutide, dulaglutide, semaglutide, pramlintide, and lixisenatide; the cytokine mimetic peptide comprises erythropoietin (EPO) and thrombopoietin (TPO); the antiviral peptide comprises thymopentin, cobicistat, and enfuvirtide; the immunosuppressive peptide comprises cyclosporin; the antimicrobial peptide comprises caspofungin, teicoplanin, and micafungin; the peptide for treating osteoporosis comprises teriparatide, calcitonin salmon, and abaloparatide; the anti-tumor peptide comprises degarelix, triptorelin, and mifamurtide; and the auxin peptide comprises sermorelin, follicle-stimulating hormone, urofollitropin, lanreotide, glutathione, and a polypeptide vaccine.

**7.** The high-permeability protein or polypeptide conjugate according to claim 1, wherein a functional group on the protein or the polypeptide is linked to a reactive group on a terminal chain or a side chain of the tertiary amine oxide group-containing polymer through a chemical bond.

**8.** The high-permeability protein or polypeptide conjugate according to claim 7, wherein the functional group comprises amino, carboxyl, hydroxyl, and sulfhydryl, and the chemical bond comprises an amide bond, a urea bond, a carbamate bond, a thiourea bond, an ester bond, and an ether bond.

**9.** The high-permeability protein or polypeptide conjugate according to claim 1, wherein a tertiary amine oxide group in the tertiary amine oxide group-containing polymer is an oxide of a saturated or unsaturated tertiary amine, and the saturated or unsaturated tertiary amine is selected from one of N,N-dimethylamino, N,N-diethylamino, N,N-dipropylamino, N,N-methylethylamino, N-pyrrolidinyl, N-piperidinyl, N-morpholinyl, and pyridyl.

**10.** The high-permeability protein or polypeptide conjugate according to claim 1, wherein the tertiary amine oxide group-containing polymer is a poly(meth)acrylate, a poly(meth)acrylamide, a polyamino acid, a polyester, or a polyethyleneimine.

**11.** The high-permeability protein or polypeptide conjugate according to claim 10, wherein the tertiary amine oxide group-containing polymer has a molecular weight of 500 Da to 50,000 Da.

**12.** The high-permeability protein or polypeptide conjugate according to claim 1, wherein the tertiary amine oxide group-containing polymer is selected from one of poly[2-(N-oxide-N,N-dimethylamino)ethyl methacrylate] (OPDMA), poly [2-(N-oxide-N,N-diethylamino)ethyl methacrylate] (OPDEA), poly[2-(N-oxide-N,N-diethylamino)ethyl acrylate] (OPDEAA), poly[N-oxide-4-vinylpyridine](OPVP), poly[glutamic acid-2-(N-oxide-N,N-dimethylamino)ethyl ester] (OPGADMe), poly[glutamic acid-2-(N-oxide-N,N-dimethylamino)acetamide] (OPGADMa), poly[glutamic acid-2-(N-oxide-N,N-diethylamino)ethyl ester] (OPGADEe), poly[glutamic acid-2-(N-oxide-N,N-diethylamino)acetamide] (OPGADEa), poly{$N^{\varepsilon}$-[2-(N-oxide-N,N-dimethylamino)ethyloxyacyl]lysine} (OPLLDMe), poly{$N^{\varepsilon}$-[2-(N-oxide-N,N-dimethylamino)ethylaminoacyl]lysine} (OPLLDMa), a polyglutamic acid (PGA) dendritic macromolecule comprising 2-(N-oxide-N,N-dimethylamino)ethyl ester (amide) on a surface, and a poly-L-lysine (PLL) dendritic macromolecule.

**13.** A use of the high-permeability protein or polypeptide conjugate according to claim 1 as a raw material for preparing a transdermal formulation, an eye drop formulation, or an oral formulation.

1/17

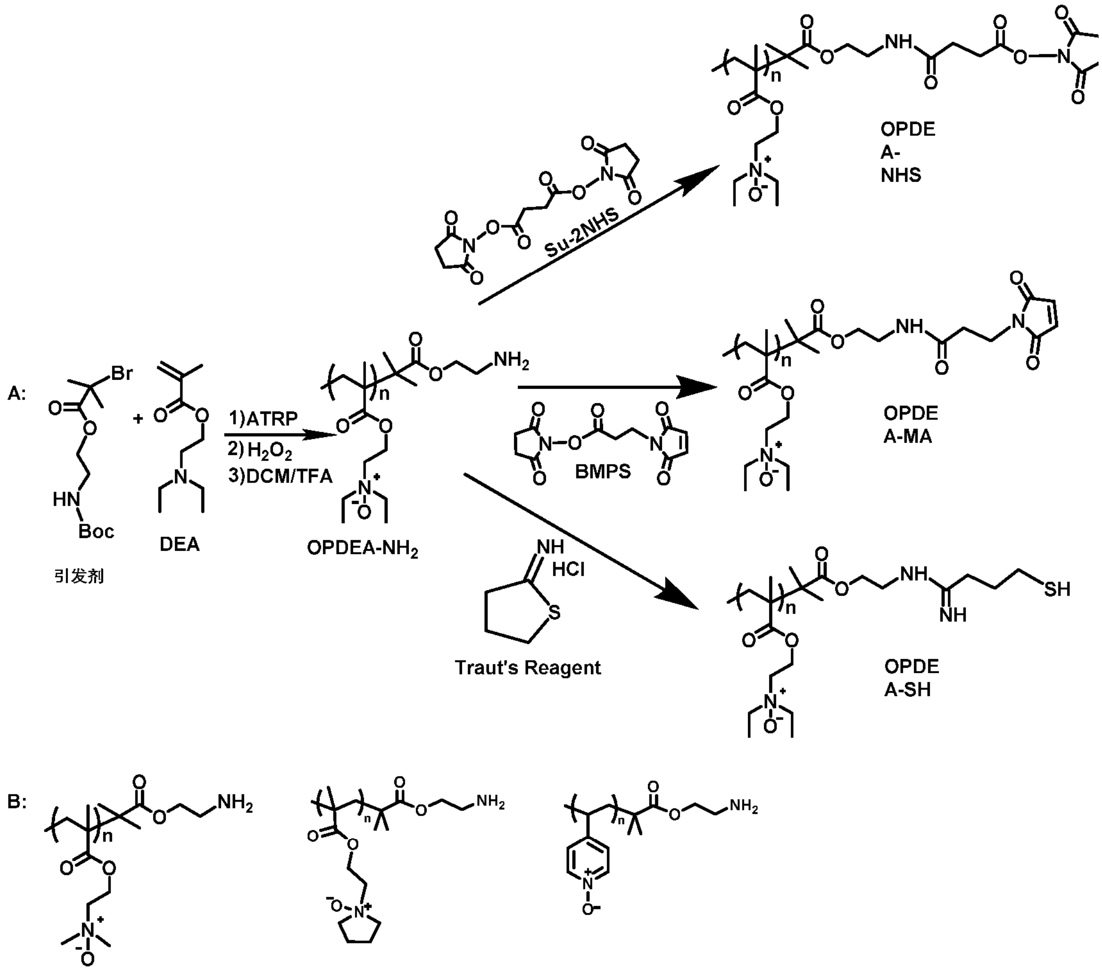


FIG. 1

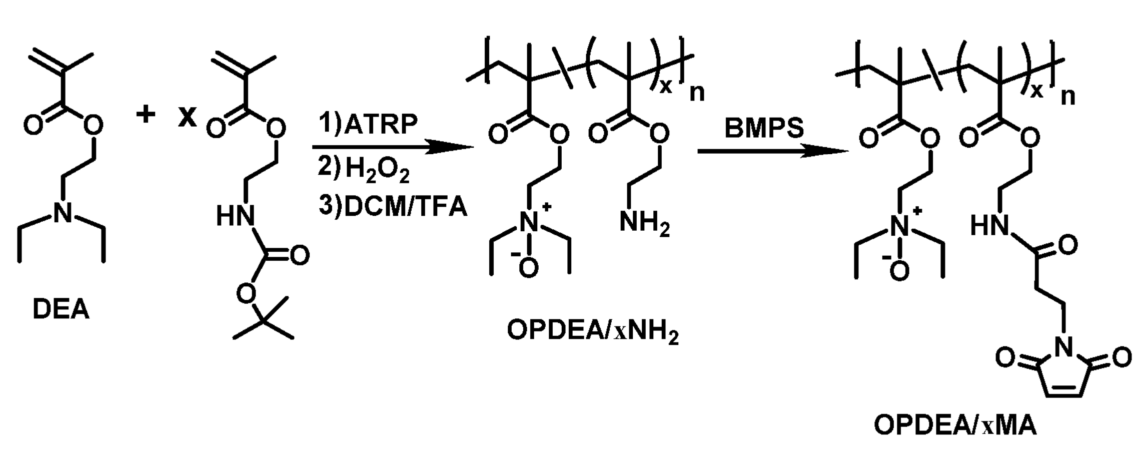


FIG. 2

A

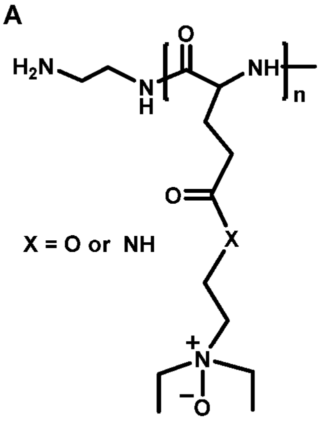

X = O or NH

X=O ester OPGAe-$NH_2$
X=NH amide OPGAa-$NH_2$

B

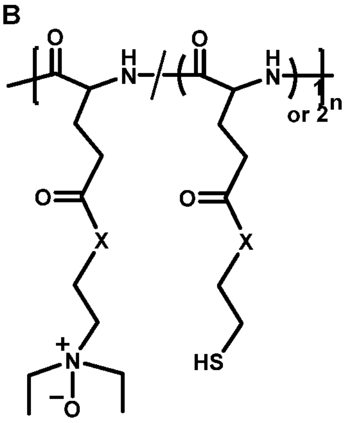

OPGA-SH

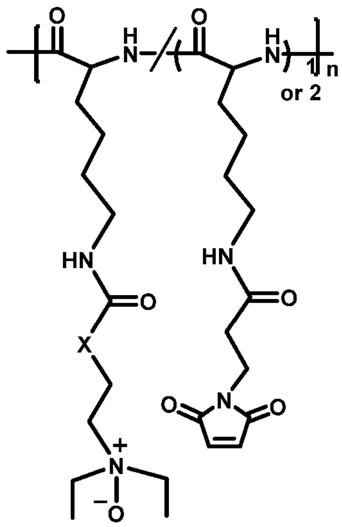

OPLL-MA

C

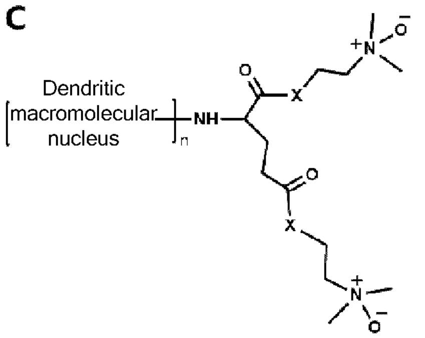

**Polyglutamic acid (PGA) dendritic macromolecule functionalized by a tertiary amine N-oxide on a surface**
X = O, Ester bond, ODPGAe
X = NH, Amide bond, ODPGAa
n is determined by a polymerization degree.

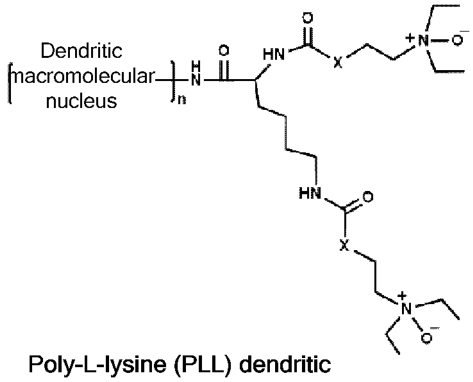

**Poly-L-lysine (PLL) dendritic macromolecule functionalized by a tertiary amine N-oxide on a surface**
X = O, Carbamate bond, ODPLLe
X = NH, Ester bond, ODPGAa
n is determined by a polymerization degree

FIG. 3

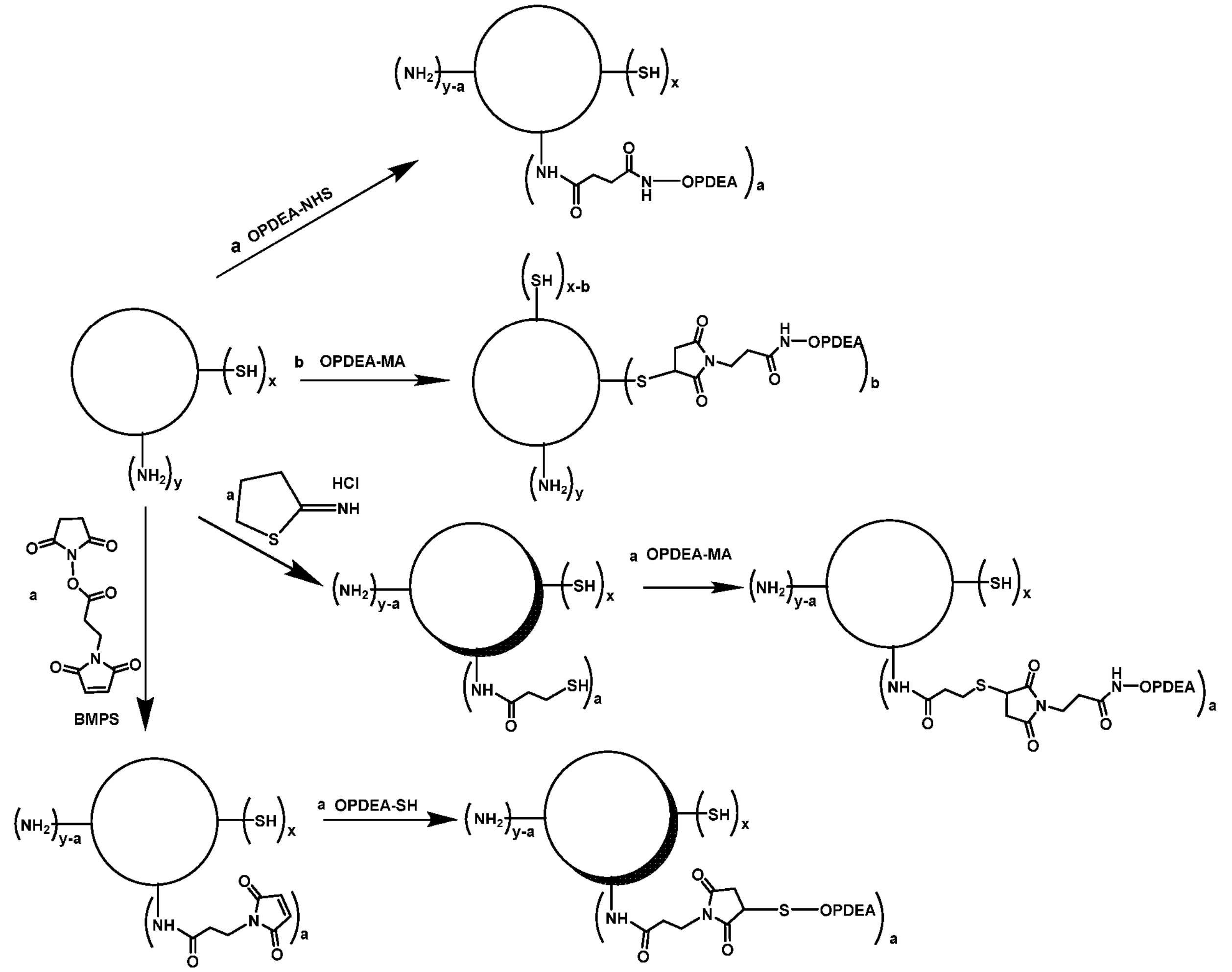


FIG. 4

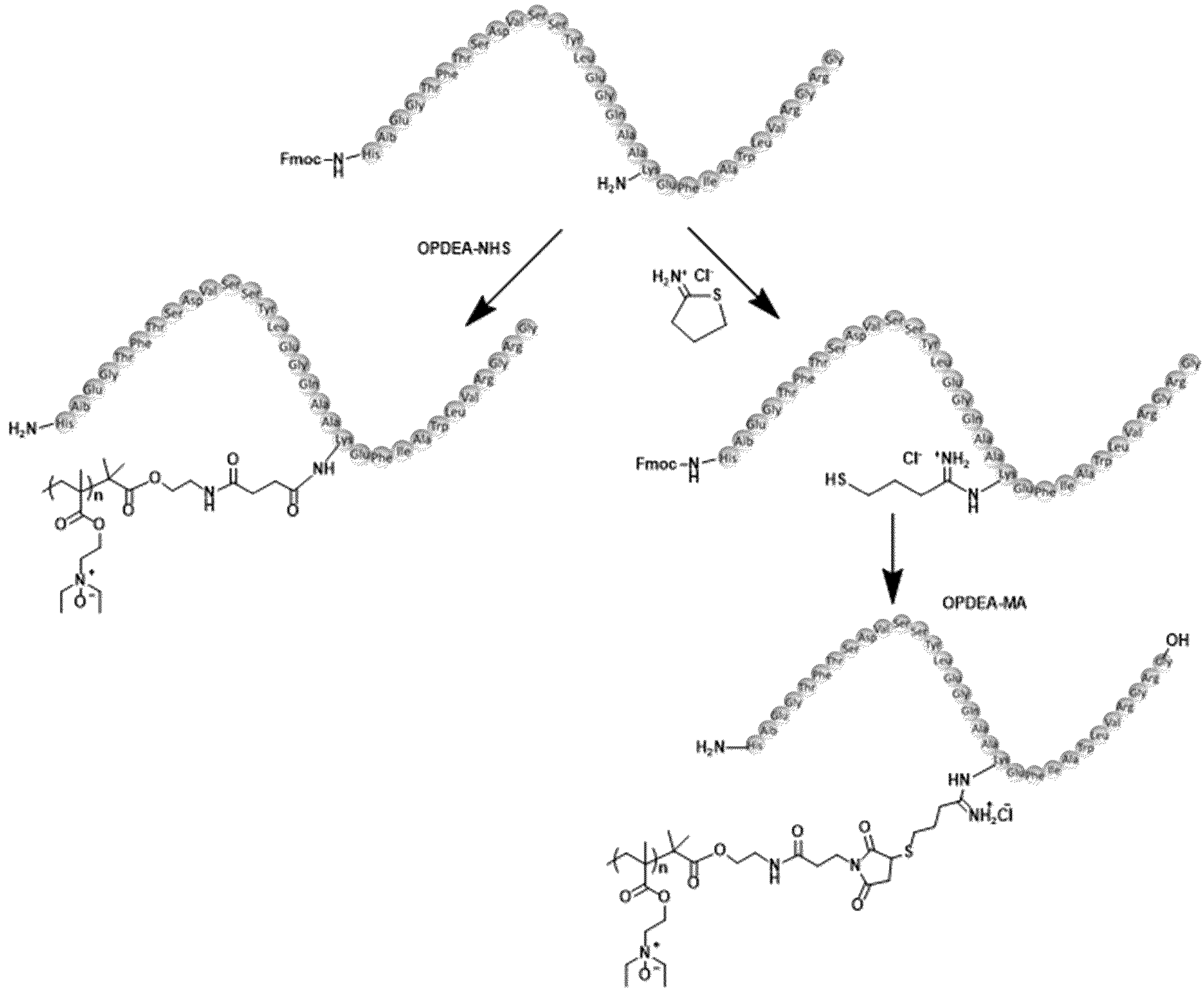


FIG. 5

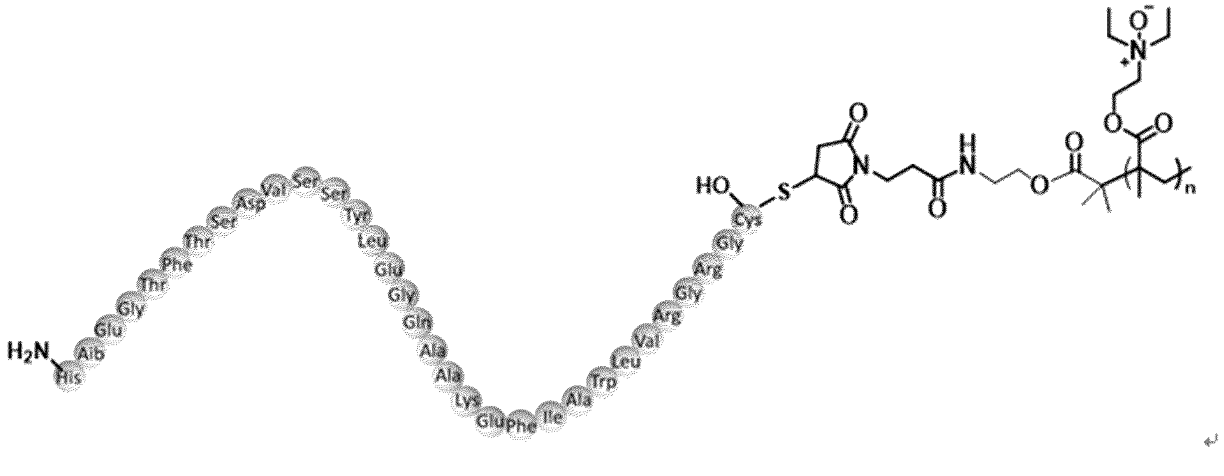

FIG. 6

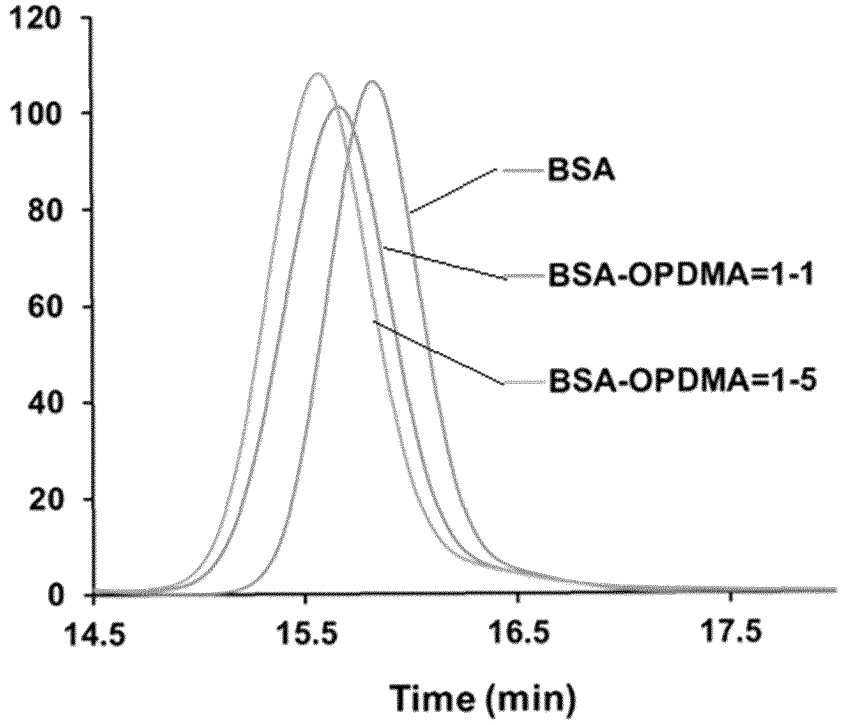
120
100
80
60
40
20
0
14.5
15.5
16.5
17.5
Time (min)
BSA
BSA-OPDMA=1-1
BSA-OPDMA=1-5

FIG. 7

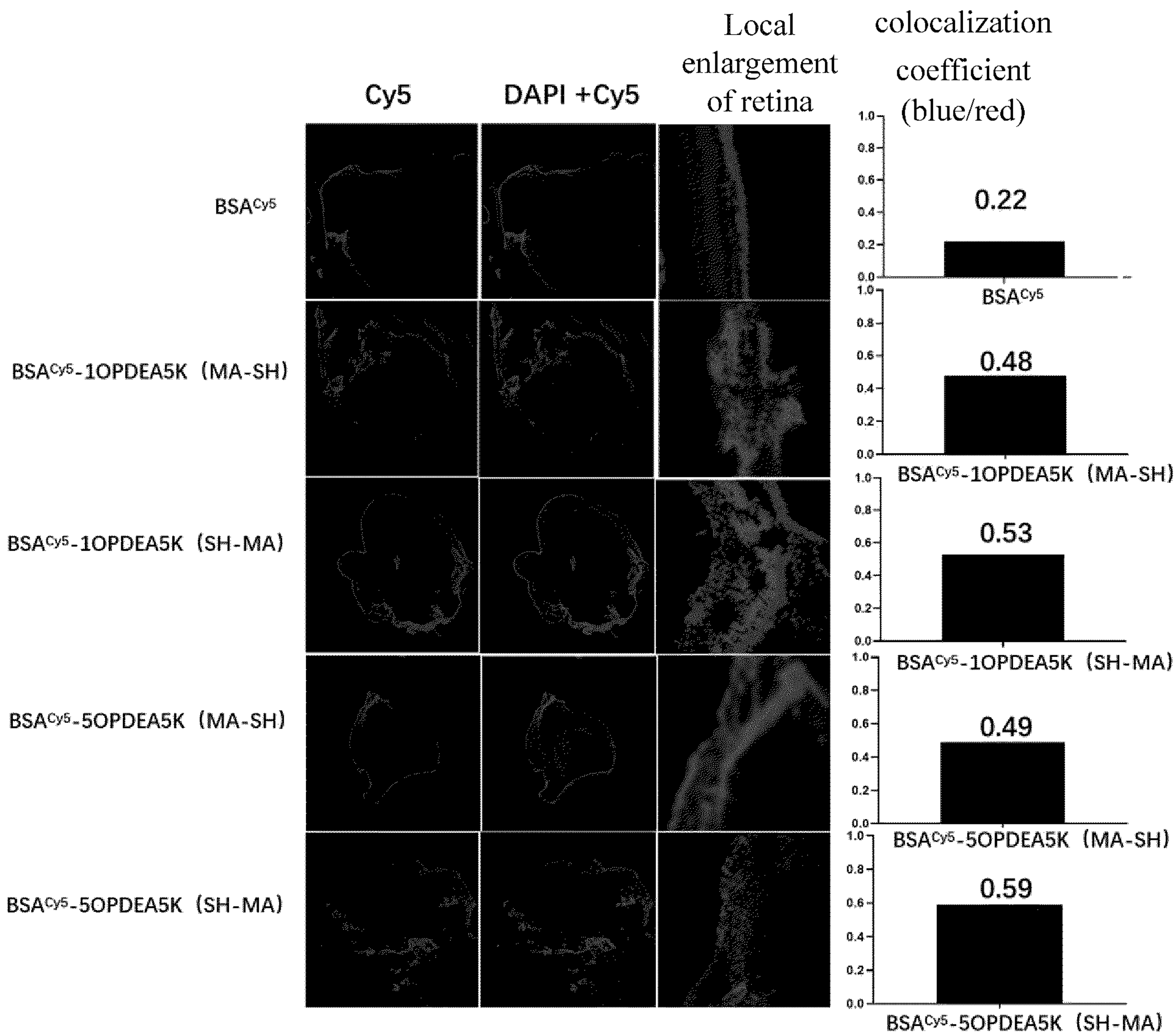
Cy5
DAPI +Cy5
Local enlargement of retina
colocalization coefficient (blue/red)
BSACy5
BSACy5-10PDEA5K (MA-SH)
BSACy5-10PDEA5K (SH-MA)
BSACy5-50PDEA5K (MA-SH)
BSACy5-50PDEA5K (SH-MA)
0.22
0.48
0.53
0.49
0.59
1.0
0.8
0.6
0.4
0.2
0.0

FIG. 8

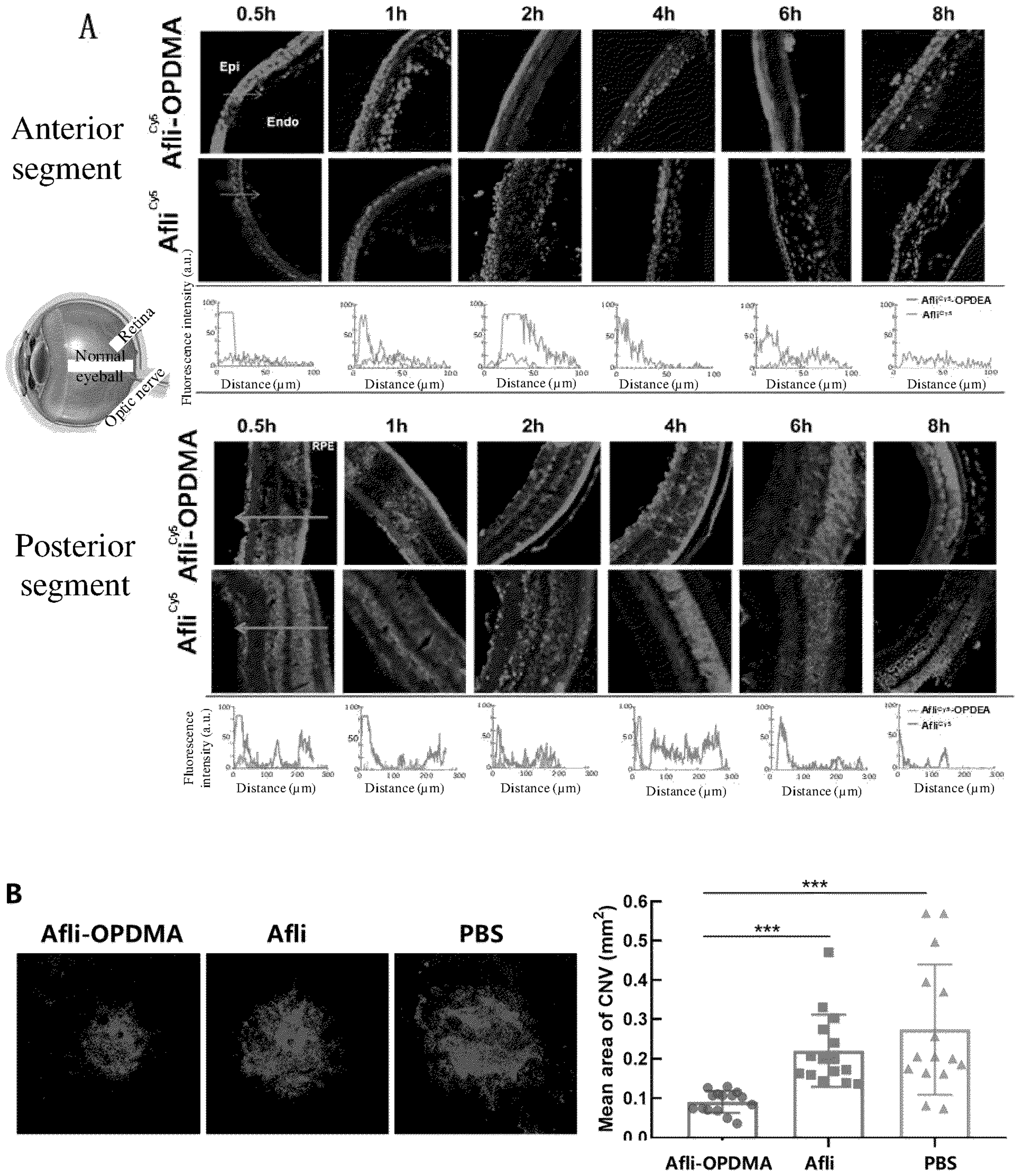
A
Anterior segment
Posterior segment
0.5h
1h
2h
4h
6h
8h
Afli$^{Cy5}$-OPDMA
Afli$^{Cy5}$
Epi
Endo
RPE
Retina
Normal eyeball
Optic nerve
Fluorescence intensity (a.u.)
Distance (μm)
Afli$^{Cy5}$-OPDEA
B
Afli-OPDMA
Afli
PBS
Mean area of CNV (mm$^2$)
***
0.6
0.5
0.4
0.3
0.2
0.1
0.0

FIG. 9

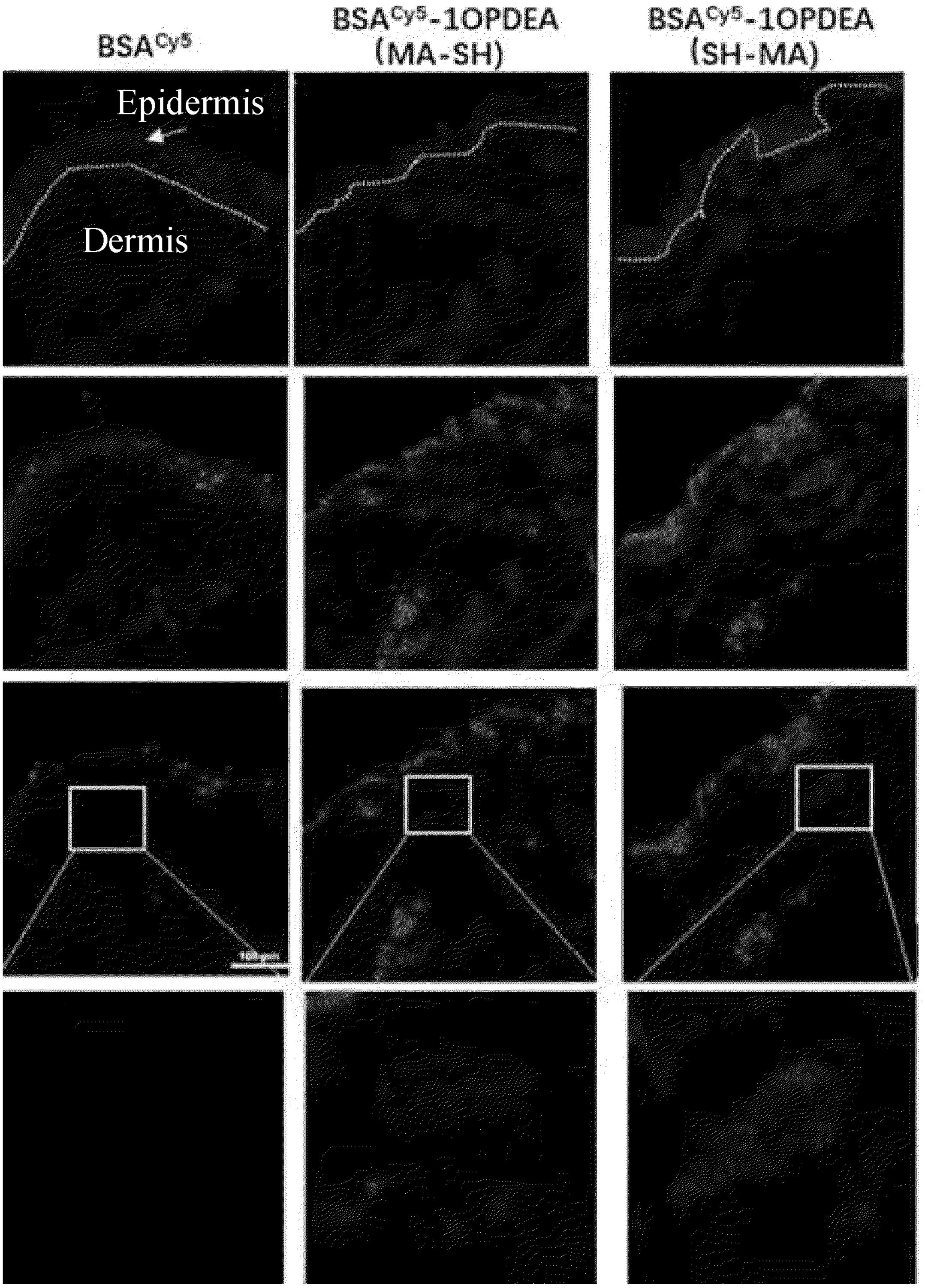
BSA$^{Cy5}$
BSA$^{Cy5}$-1OPDEA (MA-SH)
BSA$^{Cy5}$-1OPDEA (SH-MA)
Epidermis
Dermis

FIG. 10

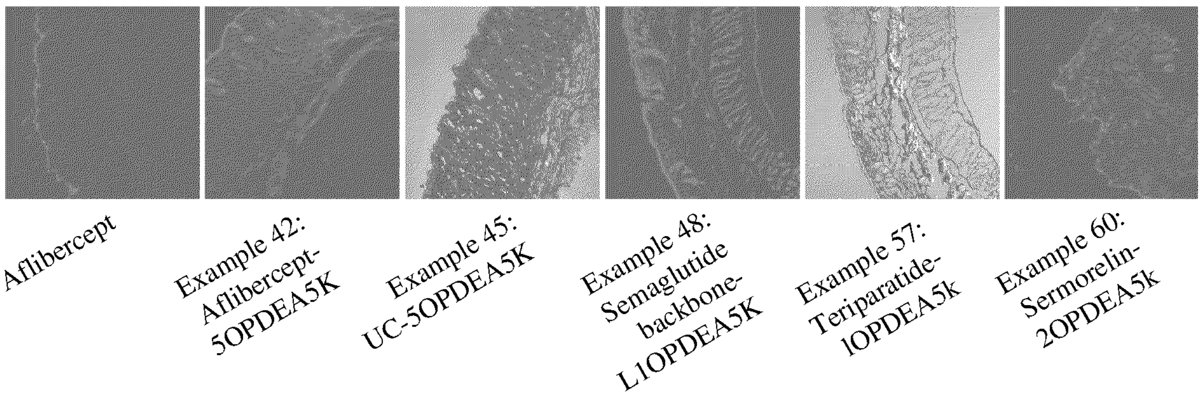
Aflibercept
Example 42:
Aflibercept-
5OPDEA5K
Example 45:
UC-5OPDEA5K
Example 48:
Semaglutide
backbone-
L1OPDEA5K
Example 57:
Teriparatide-
1OPDEA5k
Example 60:
Sermorelin-
2OPDEA5k

FIG. 11

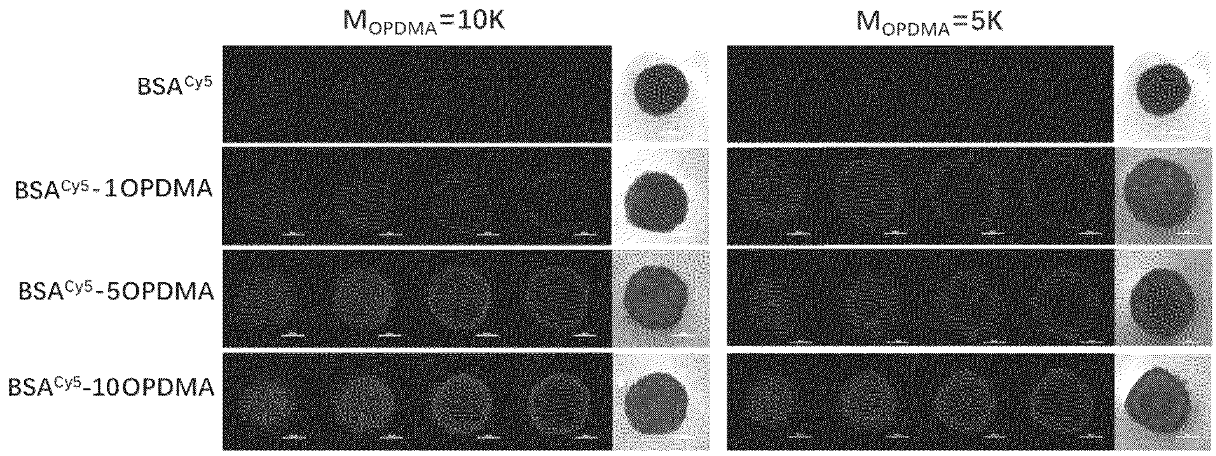
$M_{OPDMA}$=10K
$M_{OPDMA}$=5K
$BSA^{Cy5}$
$BSA^{Cy5}$-1OPDMA
$BSA^{Cy5}$-5OPDMA
$BSA^{Cy5}$-10OPDMA

FIG. 12

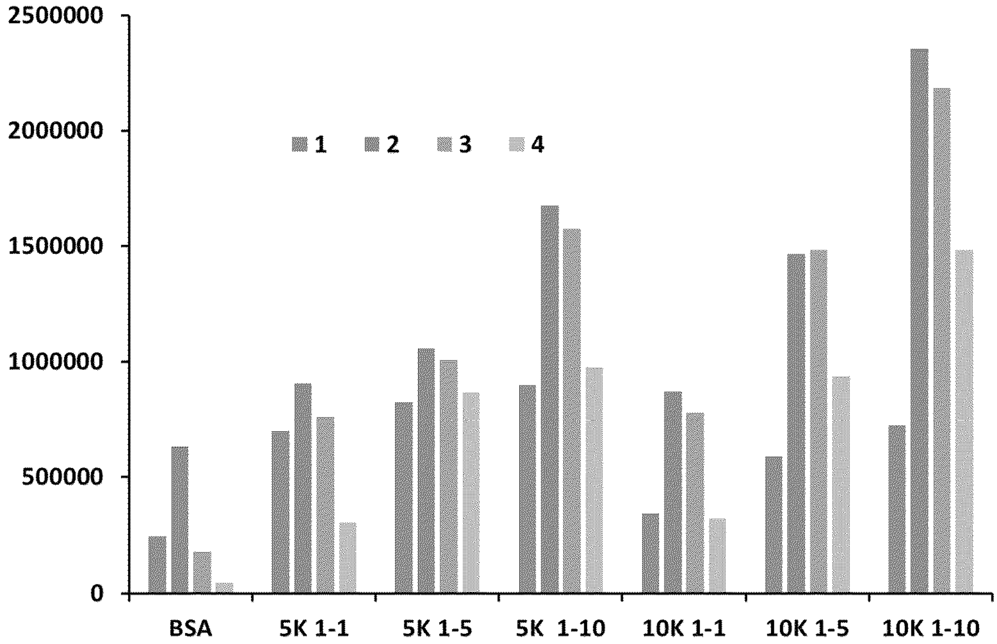
2500000
2000000
1500000
1000000
500000
0
1
2
3
4
BSA
5K 1-1
5K 1-5
5K 1-10
10K 1-1
10K 1-5
10K 1-10

FIG. 13

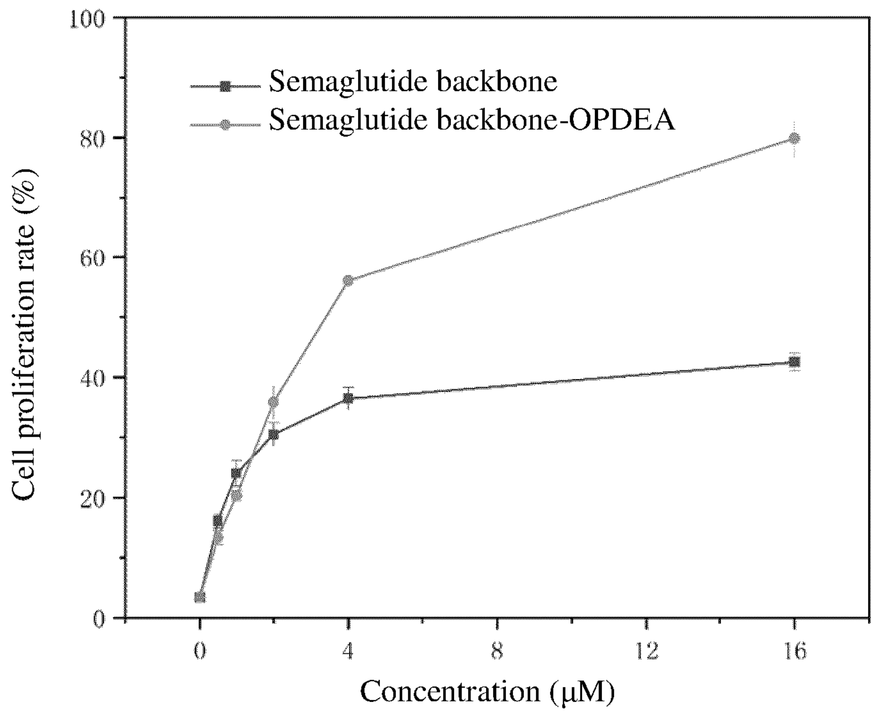
Semaglutide backbone
Semaglutide backbone-OPDEA
Cell proliferation rate (%)
Concentration (μM)
0
20
40
60
80
100
0
4
8
12
16

FIG. 14

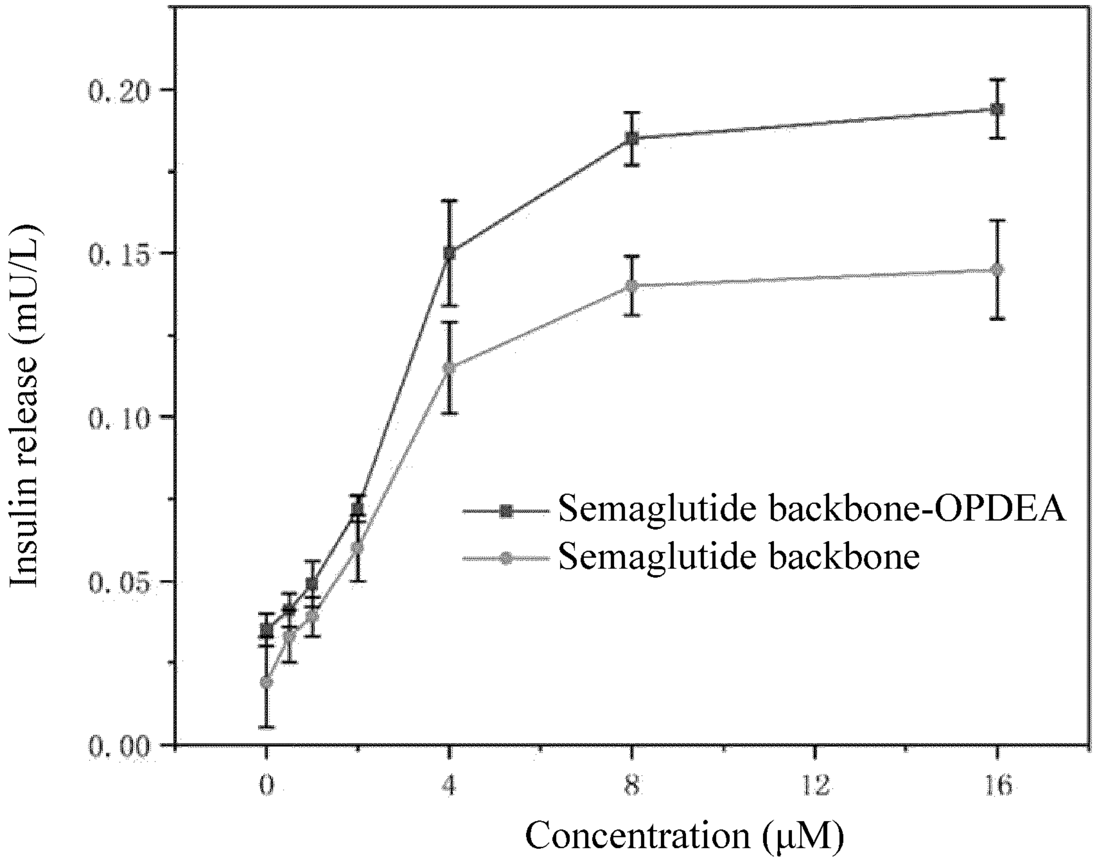
0.20
0.15
0.10
0.05
0.00
Insulin release (mU/L)
Semaglutide backbone-OPDEA
Semaglutide backbone
0
4
8
12
16
Concentration (μM)

FIG. 15

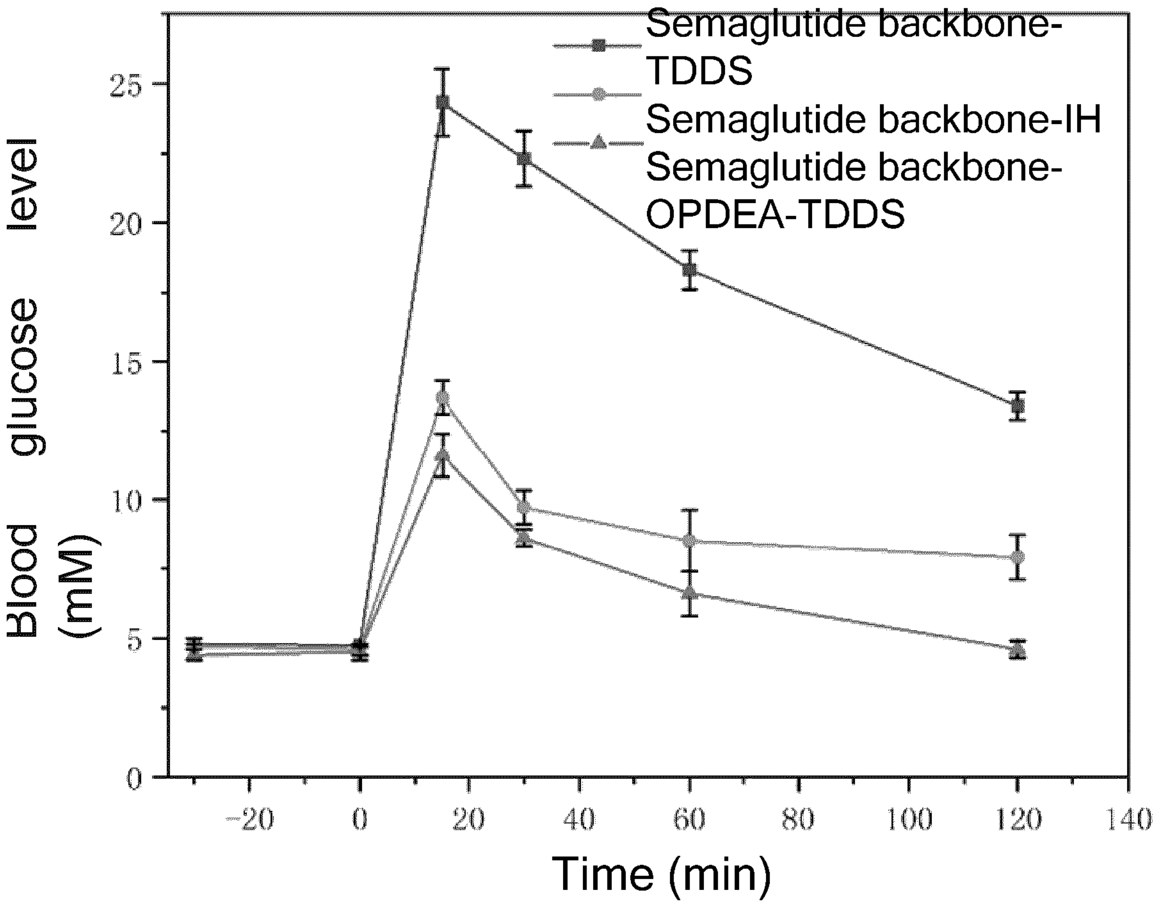
Semaglutide backbone-TDDS
Semaglutide backbone-IH
Semaglutide backbone-OPDEA-TDDS
Blood glucose level (mM)
Time (min)
0
5
10
15
20
25
-20
0
20
40
60
80
100
120
140

FIG. 16

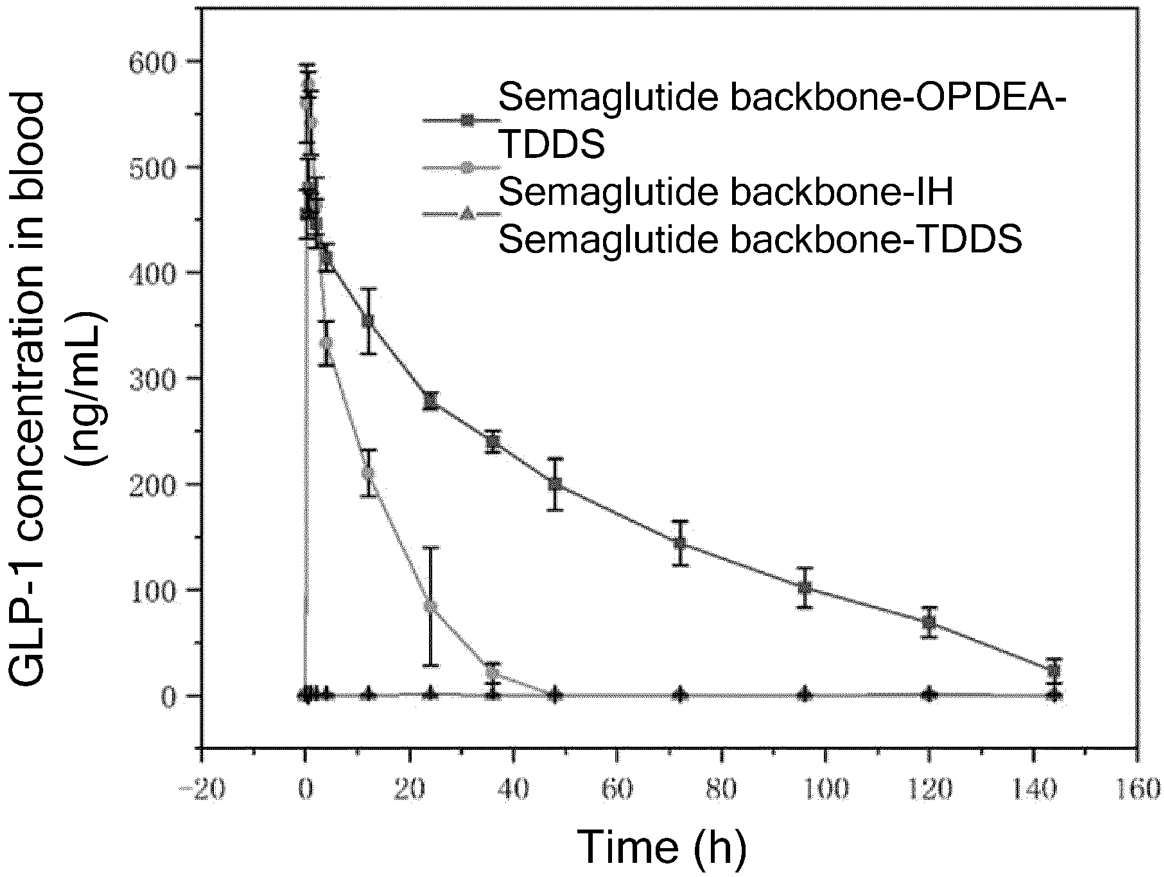
GLP-1 concentration in blood (ng/mL)
600
500
400
300
200
100
0
-20
0
20
40
60
80
100
120
140
160
Time (h)
Semaglutide backbone-OPDEA-TDDS
Semaglutide backbone-IH
Semaglutide backbone-TDDS

FIG. 17

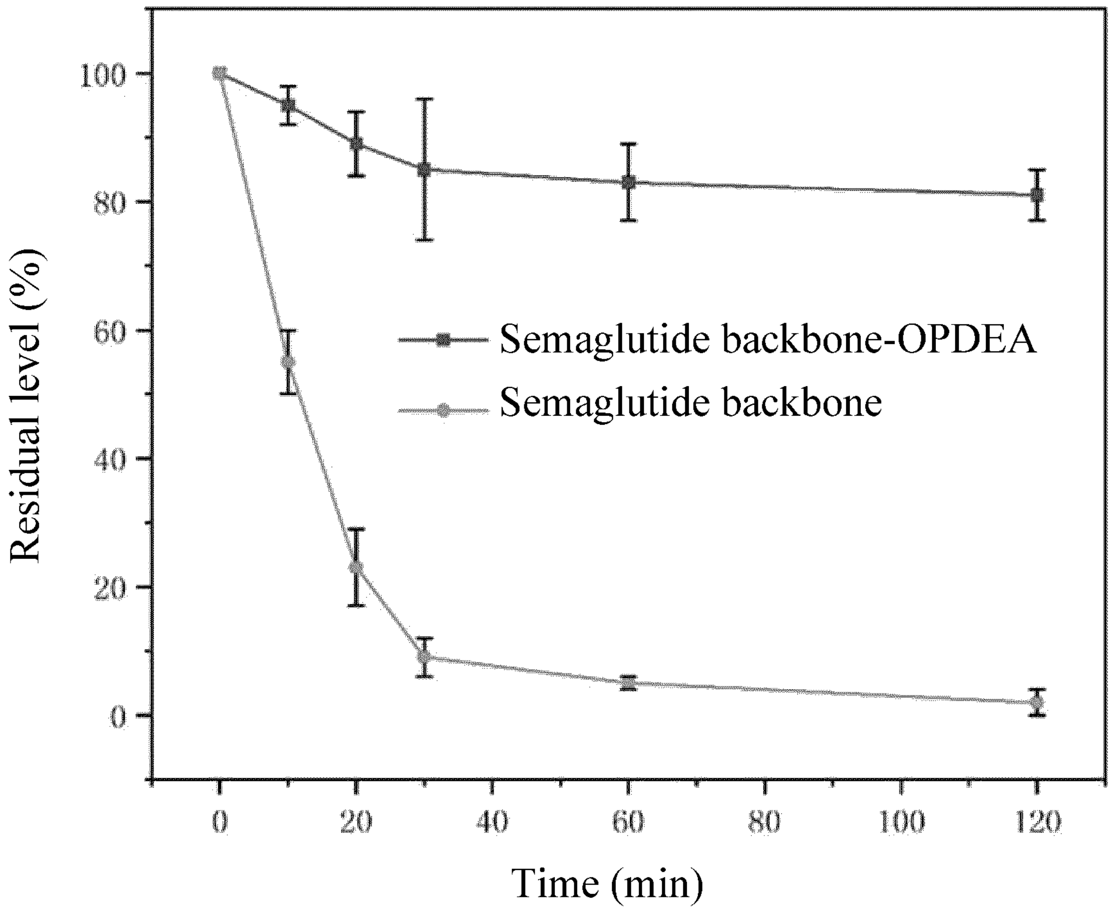
100
80
60
40
20
0
Residual level (%)
Semaglutide backbone-OPDEA
Semaglutide backbone
0
20
40
60
80
100
120
Time (min)

FIG. 18

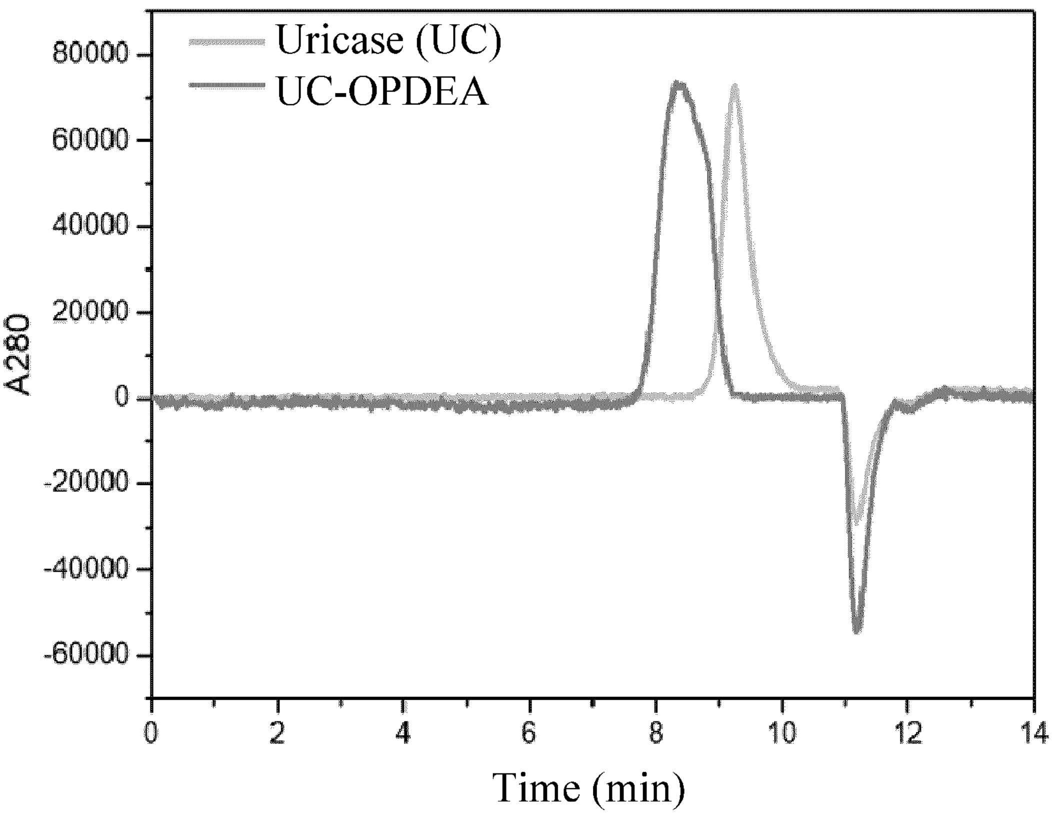
Uricase (UC)
UC-OPDEA
80000
60000
40000
20000
0
-20000
-40000
-60000
A280
0
2
4
6
8
10
12
14
Time (min)

FIG. 19

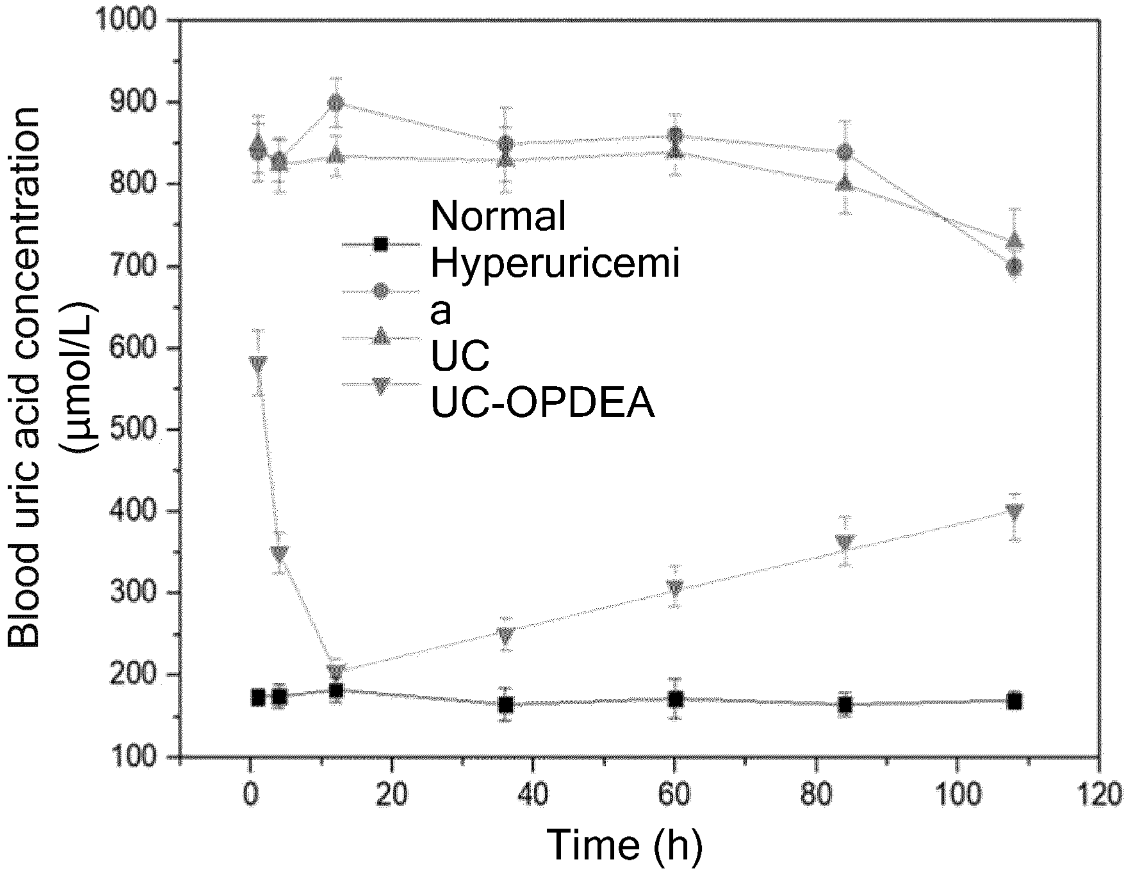
1000
900
800
700
600
500
400
300
200
100
Blood uric acid concentration (μmol/L)
Normal
Hyperuricemi
a
UC
UC-OPDEA
0
20
40
60
80
100
120
Time (h)

FIG. 20

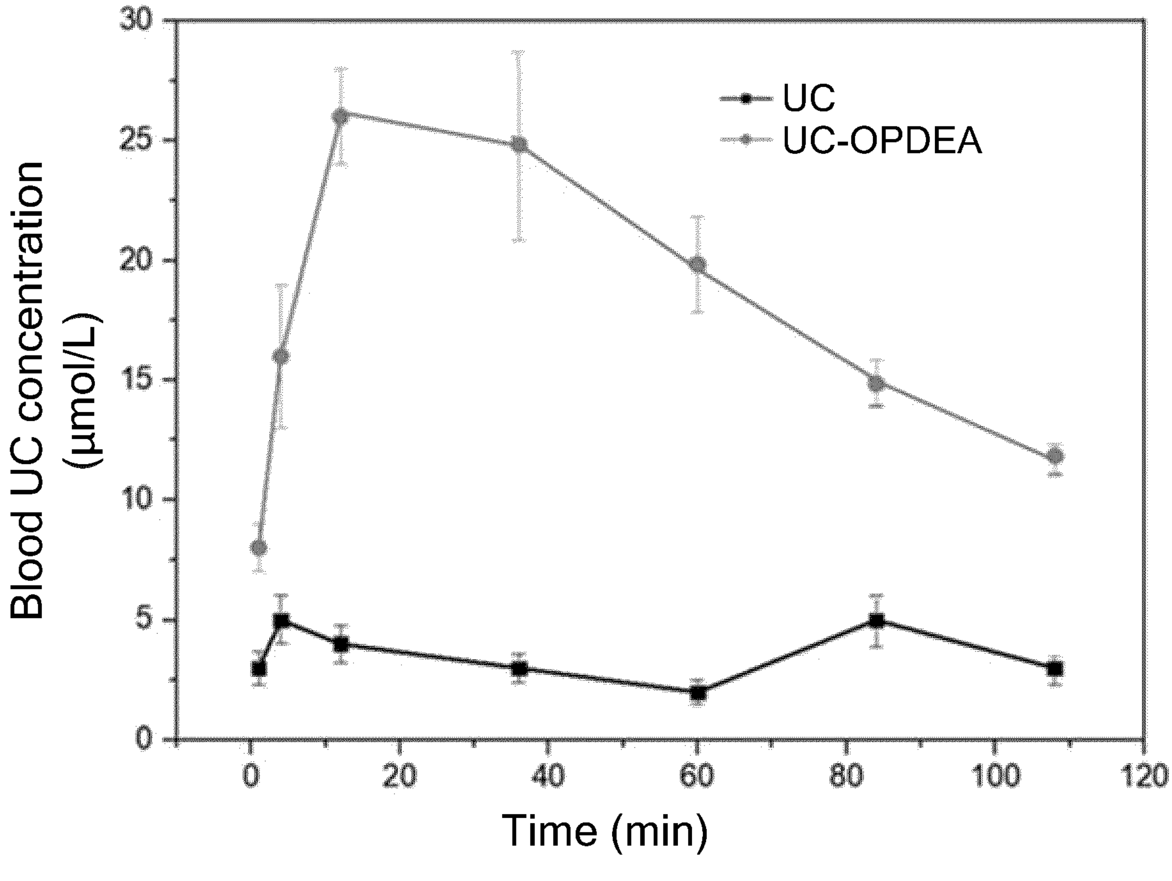
UC
UC-OPDEA
Blood UC concentration (μmol/L)
30
25
20
15
10
5
0
0
20
40
60
80
100
120
Time (min)

FIG. 21

**INTERNATIONAL SEARCH REPORT**

International application No.
**PCT/CN2023/139354**

**A. CLASSIFICATION OF SUBJECT MATTER**

A61K47/58(2017.01)i; A61K47/59(2017.01)i; A61K39/395(2006.01)i; A61K38/16(2006.01)i; A61K38/08(2019.01)i; A61K38/26(2006.01)i; A61K38/29(2006.01)i; A61K38/23(2006.01)i; A61K38/27(2006.01)i; A61K9/08(2006.01)i; A61P3/10(2006.01)i; A61P19/10(2006.01)i; A61P37/02(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CJFD, CNTXT, DWPI, ENTXTC, WPABSC, CNABS, VEN, CNKI, PUBMED, STN: 偶联, 键合, 聚, N-氧化, 三级胺, 二甲基胺基, 二乙基胺基, 氧化聚乙烯基吡啶, 氧化聚乙烯基咪唑, OPDMA, OPDEA, OPDMAA, OPDEAA, OPVP, OPGADMe, OPGADMa, OPGADEe, OPGADEa, OPLLDMe, OPLLDMa, 蛋白, 肽, 抗体, 透皮, 眼, 口服, conjugat+, bond+, N-oxide, amine-oxide, tertiary amine, dimethylamine, diethylamine, oxide, polyvinyl pyridines, polyvinyl imidazoles, protein? , peptides, antibod+, transdermal, eye, ophthal+, oral

**C. DOCUMENTS CONSIDERED TO BE RELEVANT**

| Category* | Citation of document, with indication, where appropriate, of the relevant passages | Relevant to claim No. |
|---|---|---|
| PX | CN 116617407 A (ZHEJIANG UNIVERSITY) 22 August 2023 (2023-08-22) claims 1-13 | 1-13 |
| PX | CN 116135877 A (ZHEJIANG UNIVERSITY) 19 May 2023 (2023-05-19) claims 1-14 | 1-13 |
| PX | CN 116077674 A (PEKING UNIVERSITY) 09 May 2023 (2023-05-09) claims 1-10, and embodiment 1, and figure 1 | 1-12 |
| X | CN 111479563 A (UNIVERSITY OF WASHINGTON) 31 July 2020 (2020-07-31) claims 25-27, and description, paragraphs [0010], [0067]-[0073], [0228] and [0341]-[0344] | 1-6, 9-11 |
| Y | CN 111479563 A (UNIVERSITY OF WASHINGTON) 31 July 2020 (2020-07-31) claims 25-27, and description, paragraphs [0010], [0067]-[0073], [0228] and [0341]-[0344] | 7-8, 12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 March 2024** | **05 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/139354**

**C. DOCUMENTS CONSIDERED TO BE RELEVANT**

| Category* | Citation of document, with indication, where appropriate, of the relevant passages | Relevant to claim No. |
|---|---|---|
| A | CN 111479563 A (UNIVERSITY OF WASHINGTON) 31 July 2020 (2020-07-31)<br>claims 25-27, and description, paragraphs [0010], [0067]-[0073], [0228] and [0341]-[0344] | 13 |
| X | CN 109010838 A (ZHEJIANG UNIVERSITY) 18 December 2018 (2018-12-18)<br>abstract, and description, paragraphs [0007]-[0030] | 1-6, 9-12 |
| Y | CN 109010838 A (ZHEJIANG UNIVERSITY) 18 December 2018 (2018-12-18)<br>abstract, and description, paragraphs [0007]-[0030] | 7-8 |
| A | CN 109010838 A (ZHEJIANG UNIVERSITY) 18 December 2018 (2018-12-18)<br>abstract, and description, paragraphs [0007]-[0030] | 13 |
| Y | CN 107519497 A (ZHEJIANG UNIVERSITY) 29 December 2017 (2017-12-29)<br>abstract, and claims 1-5, and description, paragraphs [0042]-[0049] | 7-8 |
| Y | GENG, Yu et al. "A neutral water-soluble mitochondria-targeting polymer"<br>*Chem. Commun.,* Vol. 55, No. 67, 22 July 2019 (2019-07-22), 10015-10018<br>ISSN: 1359-7345,<br>abstract, page 10018, left-hand column, paragraph 2 | 12 |

Form PCT/ISA/210 (second sheet) (July 2022)

**INTERNATIONAL SEARCH REPORT**
**Information on patent family members**

International application No.
**PCT/CN2023/139354**

| Patent document cited in search report | Publication date (day/month/year) | Patent family member(s) | Publication date (day/month/year) |
|---|---|---|---|
| CN 116617407 A | 22 August 2023 | None | |
| CN 116135877 A | 19 May 2023 | None | |
| CN 116077674 A | 09 May 2023 | None | |
| CN 111479563 A | 31 July 2020 | JP 2020527382 A | 10 September 2020 |
| | | WO 2019006398 A2 | 03 January 2019 |
| | | WO 2019006398 A3 | 28 February 2019 |
| | | EP 3644980 A2 | 06 May 2020 |
| | | EP 3644980 A4 | 24 March 2021 |
| | | US 2020123294 A1 | 23 April 2020 |
| | | US 11752212 B2 | 12 September 2023 |
| CN 109010838 A | 18 December 2018 | CN 109010838 B | 17 November 2020 |
| CN 107519497 A | 29 December 2017 | CN 107519497 B | 11 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201210390598
- CN 202010259644


### Non-patent literature cited in the description


- Effects of Intravitreal Injection of Anti-Vascular Endothelial Growth Factor Drugs on Retinal Blood Circulation. *Chinese Journal of Ocular Fundus Diseases*, 2020, vol. 36, 565-570
- Antibody tumor penetration: transport opposed by systemic and antigen-mediated clearance. *Advanced Drug Delivery Review*, 2008, vol. 60, 1421-34
- Tumor drug penetration measurements could be the neglected piece of the personalized cancer treatment puzzle. *Clinical Pharmacology & Therapeutics*, 2019, vol. 106, 148-163
- Enhancement of the tumor penetration of monoclonal antibody by fusion of a neuropilin-targeting peptide improves the anti-tumor efficacy. *Molecular Cancer Therapy*, 2014, vol. 13, 651-661
- Progress on the Long-Lasting Protein and peptide Drugs. *Life Sciences*, 2008, vol. 2, 258-262
- Research Progress of Polypeptides Transdermal Delivery. *Chinese Journal of Hospital Pharmacy*, 2018, vol. 19, 2084-2087
- *Nature Biomedical Engineering*, 2021, vol. 5, 1019-1037